# EUROPEAN PATENT APPLICATION

(11) **EP 4 446 317 A1**
(43) Date of publication of application: **16.10.2024**
(21) Application number: 22903528.2
(22) Date of filing: 07.12.2022
(51) Int. Cl.: C07D 401/14, A61K 31/495, A61P 35/00

(54) **SALT OF 3,4-DIHYDROISOQUINOLINE COMPOUND AND USE THEREOF**

(30) Priority: 08.12.2021 CN 202111494667
(71) Applicant: CSPC Zhongqi Pharmaceutical Technology (Shijiazhuang) Co., Ltd., Shijiazhuang, Hebei 050035 (CN)
(72) Inventor: PENG, Xiaoshi, Shijiazhuang, Hebei 050035 (CN); LI, Pengfei, Shijiazhuang, Hebei 050035 (CN); JIN, Gengen, Shijiazhuang, Hebei 050035 (CN); LI, Can, Shijiazhuang, Hebei 050035 (CN); YANG, Min, Shijiazhuang, Hebei 050035 (CN); LIU, Xiaozheng, Shijiazhuang, Hebei 050035 (CN); CUI, Qiaoli, Shijiazhuang, Hebei 050035 (CN); SI, Yajuan, Shijiazhuang, Hebei 050035 (CN)
(74) Representative: Patentanwälte Olbricht Buchhold Keulertz
(86) International application number: PCT/CN2022/137298
(87) International publication number: WO 2023/104107

(57) **Abstract**

Provided are a salt of a compound represented by formula (A), a solid form and a crystal form thereof, a pharmaceutical composition comprising same, the use thereof and a preparation method therefor. Compared with the compound represented by formula (A), a salt of the compound represented by formula (A) has higher solubility in water and a biological solvent medium, is easy to be prepared into a solid form, is convenient to transfer and weigh, and has good application prospects.

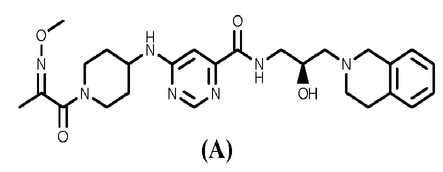

## Description

The present disclosure claims priority to the Chinese Patent Application No. 202111494667.8, filed on December 08, 2021, the entirty of which is incorporated herein by reference.

### Tchnical Field

The invention belongs to the technical field of pharmaceuticals, and relates to a salt of a 3,4-dihydroisoquinoline compound, a crystalline form thereof and particular crystal forms, a pharmaceutical composition comprising the same, and the use thereof in the pharmaceutical field.

### Background

Protein arginine methyltransferases (PRMTs) are a class of S-adenosyl methionine (SAM or AdoMet)-dependent methyltransferases, which are enzymes capable of catalyzing methylation reaction of protein arginine. Specifically, they are responsible for transferring a methyl group from the AdoMet to a guanidino nitrogen atom at the terminal of arginine residue in histone or additional protein. PRMTs play important roles in the methylation of proteins, for example, participation in alternative splicing, post-transcriptional regulation, RNA processing, cell proliferation, cell differentiation, apoptosis, tumor formation, etc. According to different ways to catalyze the methylation of arginine, the members of PRMT family may be divided into three types: PRMT1-4, PRMT6, and PRMT8 belong to type I for the catalytic types of monomethylation and asymmetric dimethylation; PRMT5 and PRMT9 belong to type II for the catalytic type of symmetric dimethylation; and PRMT7 belongs to type III, capable of catalyzing the monomethylation.

PRMT5 has been isolated for the first time by Pollack *et al.* from a protein complex bound to Jak2 (Janus tyrosine kinase 2) in the yeast two-hybrid assay, so it is also known as JBP1 (jak-binding protein 1). PRMT5 can not only regulate the process of gene transcription and protein modification, but also play roles in regulating cell proliferation, differentiation, and apoptosis during the growth of tumor cells. It is a tumor therapeutic target of great potential. To date, the researches and developments of PRMT5 inhibitors are all in their early stages, of which GSK3326595 released by GSK has advanced most rapidly, which is in Phase VII clinical trial. JNJ-64619178 released for the first time by Janssen, PF-06939999 released by Pfizer, and PRT-543 released by Prelude Therapeutics are all in Phase I clinical trial. At present, the structural formulae of PF-06939999 and PRT-543 have not yet been published. The structural formulae of GSK3326595 and JNJ-64619178 are as follows:

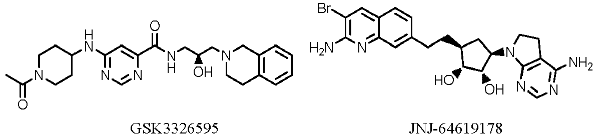

Given that no PRMT5 inhibitor has currently been approved for marketing, it would be of significant value of clinical application to design and synthesize a novel PRMT5 inhibitor having both good efficacy and good performance of administration.

### Summary of the Invention

In a first aspect, the present disclosure provides a salt of a compound represented by formula (A): 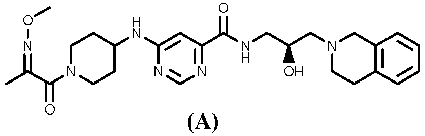 wherein the salt is an inorganic acid addition salt or an organic acid addition salt.

According to some embodiments of the present disclosure, the inorganic acid addition salt is a sulfate or phosphate.

According to some embodiments of the present disclosure, the organic acid addition salt is selected from a malate, an oxalate, a succinate, a tartrate, an adipate, a citrate, a gluconate, a maleate, a fumarate, a lactate and a gentisate; preferably a malate, an oxalate, a succinate, an L-tartrate, an L-lactate, an adipate, a citrate or a gluconate; preferably a malate, an oxalate, a succinate, an L-tartrate, an adipate, a citrate or a gluconate; further preferably a malate, an oxalate, a citrate or a gluconate; still further preferably a malate or an oxalate; still further preferably an L-malate or an oxalate.

According to some embodiments of the present disclosure, in the salt of the compound represented by formula (A), the stoichiometric ratio of the compound represented by formula (A) to the organic acid or inorganic acid molecule is 1:0.5-2; preferably 1:1-1.5; further preferably 1:1, 1:1.1, 1:1.2, 1:1.3, 1:1.4 or 1:1.5; further preferably 1:1, 1:1.3, 1:1.4 or 1:1.5; further preferably 1:1 or 1:1.5; further preferably 1:1.

According to some embodiments of the present disclosure, the compound represented by formula (A) is a compound represented by formula (A-1): 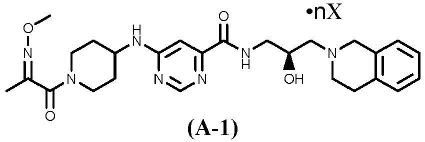 wherein X is an inorganic acid or an organic acid, and n is selected from 0.5-2; preferably 1-1.5; further preferably 1, 1.1, 1.2, 1.3, 1.4 or 1.5; further preferably 1, 1.3, 1.4 or 1.5; further preferably 1:1 or 1:1.5; still further preferably 1.

According to some embodiments of the present disclosure, X is an inorganic acid selected from sulfuric acid and phosphoric acid.

According to some embodiments of the present disclosure, X is an organic acid selected from malic acid, oxalic acid, succinic acid, tartaric acid, adipic acid, citric acid, gluconic acid, maleic acid, fumaric acid, lactic acid and gentisic acid; preferably malic acid, oxalic acid, succinic acid, L-tartaric acid, L-lactic acid, adipic acid, citric acid or gluconic acid; preferably malic acid, oxalic acid, succinic acid, L-tartaric acid, adipic acid, citric acid or gluconic acid; further preferably malic acid, oxalic acid, citric acid or gluconic acid; still further preferably malic acid or oxalic acid; still further preferably L-malic acid or oxalic acid.

According to some embodiments of the present disclosure, the salt of the compound represented by formula (A) or the compound represented by formula (A-1) is in a solid form.

According to some embodiments of the present disclosure, the solid form of the salt of the compound represented by formula (A) or the compound represented by formula (A-1) is a crystalline form.

According to some embodiments of the present disclosure, the compound represented by formula (A-1) is a compound represented by formula (B): 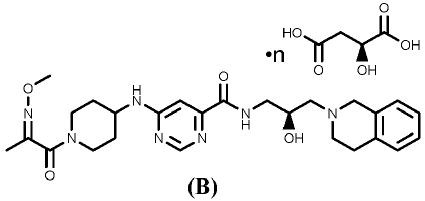 wherein n is selected from 0.5-2; preferably 1-1.5; further preferably 1, 1.1, 1.2, 1.3, 1.4 or 1.5; still further preferably 1 or 1.5.

According to some embodiments of the present disclosure, the compound represented by formula (B) is in a solid form.

According to some embodiments of the present disclosure, the solid form of the compound represented by formula (B), using KBr pellet method, has an infrared spectrum comprising characteristic peaks at the following positions (±4 cm⁻¹): 3301, 2940, 1611, 1528, 1456, 1368, 1045.

According to some embodiments of the present disclosure, the solid form of the compound represented by formula (B) is a crystalline form.

According to some embodiments of the present disclosure, the compound represented by formula (B) is a compound represented by formula (B-1):

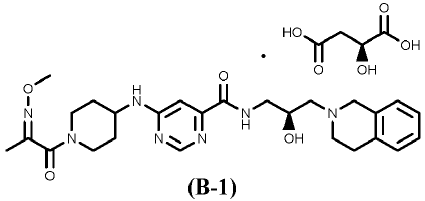

According to some embodiments of the present disclosure, the compound represented by formula (B-1) is in a solid form.

According to some embodiments of the present disclosure, the solid form of the compound represented by formula (B-1) is a crystalline form.

According to some embodiments of the present disclosure, the crystalline form of the compound represented by formula (B) is a Crystal Form I which by Cu-K_{α} radiation, has an X-ray powder diffraction pattern that comprises characteristic diffraction peaks at 2θ angles (±0.2°) of 4.2, 6.5, 13.3, and 19.1.

According to some embodiments of the present disclosure, the Crystal Form I, by Cu-K_{α} radiation, has an X-ray powder diffraction pattern that comprises characteristic diffraction peaks at 2θ angles (±0.2°) of 4.2, 6.5, 13.3, 19.1, 20.1, and 22.0.

According to some embodiments of the present disclosure, the Crystal Form I, by Cu-K_{α} radiation, has an X-ray powder diffraction pattern that comprises characteristic diffraction peaks at 2θ angles (±0.2°) of 4.2, 6.5, 13.3, 18.3, 19.1, 20.1, and 22.0.

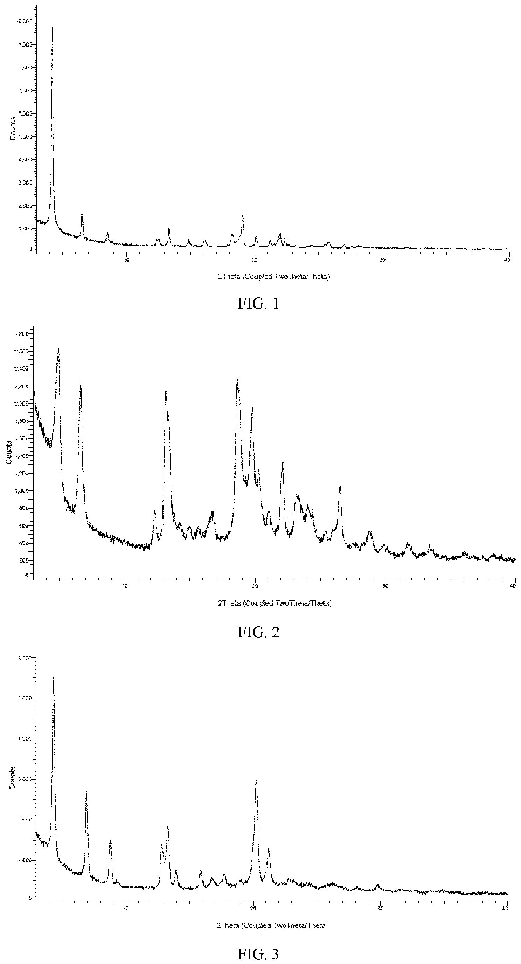

According to some embodiments of the present disclosure, the Crystal Form I, by Cu-K_{α} radiation, has an X-ray powder diffraction pattern substantially as shown in FIG. 1.

According to some embodiments of the present disclosure, the Crystal Form I has a differential scanning calorimetric curve having an endothermic peak at 113±5°C.

According to some embodiments of the present disclosure, the Crystal Form I has a differential scanning calorimetric curve having endothermic peaks at 102.15±5°C and 113±5°C.

According to some embodiments of the present disclosure, the Crystal Form I has a thermogravimetric analysis curve having a weight loss of 8.6522%±0.2% between room temperature and 110±5°C.

According to some embodiments of the present disclosure, the crystalline form of the compound represented by formula (B) is a Crystal Form II which, by Cu-K_{α} radiation, has an X-ray powder diffraction pattern that comprises characteristic diffraction peaks at 2θ angles (±0.2°) of 4.9, 6.6, 13.2, 18.7, and 19.8.

According to some embodiments of the present disclosure, the Crystal Form II, by Cu-K_{α} radiation, has an X-ray powder diffraction pattern that comprises characteristic diffraction peaks at 2θ degrees (±0.2°) of 4.9, 6.6, 13.2, 18.7, 19.8, 22.1, and 26.5.

According to some embodiments of the present disclosure, the Crystal Form II, by Cu-K_{α} radiation, has an X-ray powder diffraction pattern that comprises characteristic diffraction peaks at 2θ degrees (±0.2°) of 4.9, 6.6, 13.2, 18.7, 19.8, 22.1, 23.3, and 26.5.

According to some embodiments of the present disclosure, the Crystal Form II, by Cu-K_{α} radiation, has an X-ray powder diffraction pattern that comprises characteristic diffraction peaks at 2θ degrees (±0.2°) of 4.9, 6.6, 13.2, 18.7, 19.8, 20.3, 22.1, 23.3, and 26.5.

According to some embodiments of the present disclosure, the Crystal Form II, by Cu-K_{α} radiation, has an X-ray powder diffraction pattern that comprises characteristic diffraction peaks at 2θ degrees (±0.2°) of 4.9, 6.6, 12.3, 13.2, 18.7, 19.8, 20.3, 22.1, 23.3, 24.0, and 26.5.

According to some embodiments of the present disclosure, the Crystal Form II, by Cu-K_{α} radiation, has an X-ray powder diffraction pattern that comprises characteristic diffraction peaks at 2θ degrees (±0.2°) of 4.9, 6.6, 12.3, 13.2, 18.7, 19.8, 20.3, 22.1, 23.3, 24.0, 26.5, and 28.8.

According to some embodiments of the present disclosure, the Crystal Form II, by Cu-K_{α} radiation, has an X-ray powder diffractogram substantially as shown in FIG.2.

According to some embodiments of the present disclosure, the Crystal Form II has a differential scanning calorimetric curve having an endothermic peak at 103.58±5°C.

According to some embodiments of the present disclosure, the Crystal Form II has a differential scanning calorimetric curve having endothermic peaks at 89.8±5°C and 103.58±5°C.

According to some embodiments of the present disclosure, the Crystal Form II has a thermogravimetric analysis curve having a weight loss of 2.6358%±0.2% between room temperature and 80±5°C.

According to some embodiments of the present disclosure, the crystalline form of the compound represented by formula (B) is a Crystal Form III which, by Cu-K_{α} radiation, has an X-ray powder diffractogram that comprises characteristic diffraction peaks at 2θ degrees (±0.2) of 4.3, 6.9, and 20.3.

According to some embodiments of the present disclosure, the Crystal Form III, by Cu-K_{α} radiation, has an X-ray powder diffractogram that comprises characteristic diffraction peaks at 2θ angles (±0.2°) of 4.3, 6.9, 8.8, 20.3, and 21.2.

According to some embodiments of the present disclosure, the Crystal Form III, by Cu-K_{α} radiation, has an X-ray powder diffractogram that comprises characteristic diffraction peaks at 2θ angles (±0.2°) of 4.3, 6.9, 8.8, 12.8, 13.3, 20.3, and 21.2.

According to some embodiments of the present disclosure, the Crystal Form III, by Cu-K_{α} radiation, has an X-ray powder diffraction pattern that comprises characteristic diffraction peaks at 2θ angles (±0.2°) of 4.3, 6.9, 8.8, 12.8, 13.3, 14.0, 15.9, 20.3, and 21.2.

According to some embodiments of the present disclosure, the Crystal Form III, by Cu-K_{α} radiation, has an X-ray powder diffraction pattern substantially as shown in FIG. 3.

According to some embodiments of the present disclosure, the Crystal Form III has a differential scanning calorimetric curve having an endothermic peak at 117.93±5°C.

According to some embodiments of the present disclosure, the Crystal Form III has a differential scanning calorimetric curve having endothermic peaks at 79.82±5°C and 117.93±5°C.

According to some embodiments of the present disclosure, the Crystal Form III has a thermogravimetric analysis curve having a weight loss of 3.0001%±0.2% between room temperature and 75±5°C.

According to some embodiments of the present disclosure, the crystalline form of the compound represented by formula (B) is a Crystal Form IV which, by Cu-K_{α} radiation, has an X-ray powder diffraction pattern that comprises characteristic diffraction peaks at 2θ angles (±0.2°) of 4.4, 7.6, 8.9, 13.9, and 20.6.

According to some embodiments of the present disclosure, the Crystal Form IV, by Cu-K_{α} radiation, has an X-ray powder diffraction pattern that comprises characteristic diffraction peaks at 2θ angles (±0.2°) of 4.4, 7.6, 8.9, 12.1, 13.9, 15.2, and 20.6.

According to some embodiments of the present disclosure, the Crystal Form IV, by Cu-K_{α} radiation, has an X-ray powder diffraction pattern that comprises characteristic diffraction peaks at 2θ angles (±0.2°) of 4.4, 7.6, 8.9, 12.1, 13.9, 15.2, 17.8, 18.5, and 20.6.

According to some embodiments of the present disclosure, the Crystal Form IV, by Cu-K_{α} radiation, has an X-ray powder diffraction pattern that comprises characteristic diffraction peaks at 2θ angles (±0.2°) of 4.4, 7.6, 8.9, 12.1, 13.9, 15.2, 17.1, 17.8, 18.5, and 20.6.

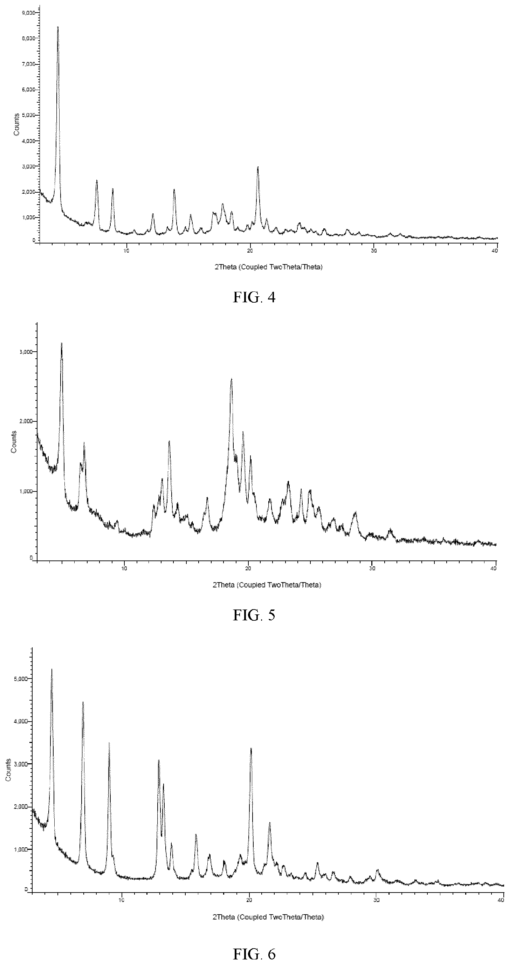

According to some embodiments of the present disclosure, the Crystal Form IV, by Cu-K_{α} radiation, has an X-ray powder diffraction pattern substantially as shown in FIG. 4.

According to some embodiments of the present disclosure, the Crystal Form IV has a differential scanning calorimetric curve having an endothermic peak at 113.27±5°C.

According to some embodiments of the present disclosure, the Crystal Form IV has a differential scanning calorimetric curve having endothermic peaks at 77.91±5°C and 113.27±5°C.

According to some embodiments of the present disclosure, the Crystal Form IV has a thermogravimetric analysis curve having a weight loss of 2.6271%±0.2% between room temperature and 60±5°C.

According to some embodiments of the present disclosure, the crystalline form of the compound represented by formula (B) is a Crystal Form V which, by Cu-K_{α} radiation, has an X-ray powder diffraction pattern that comprises characteristic diffraction peaks at 2θ angles (±0.2°) of 5.0, 13.6, 18.6, 19.6, and 20.2.

According to some embodiments of the present disclosure, the Crystal Form V, by Cu-K_{α} radiation, has an X-ray powder diffraction pattern that comprises characteristic diffraction peaks at 2θ angles (±0.2°) of 5.0, 6.8, 13.1, 13.6, 18.6, 19.6, and 20.2.

According to some embodiments of the present disclosure, the Crystal Form V, by Cu-K_{α} radiation, has an X-ray powder diffraction pattern that comprises characteristic diffraction peaks at 2θ angles (±0.2°) of 5.0, 6.8, 13.1, 13.6, 16.7, 18.6, 19.6, 20.2, and 24.3.

According to some embodiments of the present disclosure, the Crystal Form V, by Cu-K_{α} radiation, has an X-ray powder diffraction pattern that comprises characteristic diffraction peaks at 2θ angles (±0.2°) of 5.0, 6.8, 13.1, 13.6, 16.7, 18.6, 19.6, 20.2, 23.2, 24.3, and 25.0.

According to some embodiments of the present disclosure, the Crystal Form V, by Cu-K_{α} radiation, has an X-ray powder diffraction pattern that comprises characteristic diffraction peaks at 2θ angles (±0.2°) of 5.0, 6.8, 13.1, 13.6, 16.7, 18.6, 19.6, 20.2, 23.2, 24.3, 25.0, and 28.6.

According to some embodiments of the present disclosure, the Crystal Form V, by Cu-K_{α} radiation, has an X-ray powder diffraction pattern substantially as shown in FIG. 5.

According to some embodiments of the present disclosure, the Crystal Form V has a differential scanning calorimetric curve having an endothermic peak at 104.69±5°C.

According to some embodiments of the present disclosure, the Crystal Form V has a differential scanning calorimetric curve having endothermic peaks at 68.38±5°C and 104.69±5°C.

According to some embodiments of the present disclosure, the Crystal Form V has a thermogravimetric analysis curve having a weight loss of 3.6041%±0.2% between room temperature and 75±5°C.

According to some embodiments of the present disclosure, the crystalline form of the compound represented by formula (B) is a Crystal Form VI which, by Cu-K_{α} radiation, has an X-ray powder diffraction pattern that comprises characteristic diffraction peaks at 2θ angles (±0.2°) of 4.5, 7.0, 9.0, 12.9, 20.2, and 21.6.

According to some embodiments of the present disclosure, the Crystal Form VI, by Cu-K_{α} radiation, has an X-ray powder diffraction pattern that comprises characteristic diffraction peaks at 2θ angles (±0.2°) of 4.5, 7.0, 9.0, 12.9, 13.3, 15.8, 20.2, and 21.6.

According to some embodiments of the present disclosure, the Crystal Form VI, by Cu-K_{α} radiation, has an X-ray powder diffraction pattern that comprises characteristic diffraction peaks at 2θ angles (±0.2°) of 4.5, 7.0, 9.0, 12.9, 13.3, 13.9, 15.8, 20.2, and 21.6.

According to some embodiments of the present disclosure, the Crystal Form VI, by Cu-K_{α} radiation, has an X-ray powder diffraction pattern that comprises characteristic diffraction peaks at 2θ angles (±0.2°) of 4.5, 7.0, 9.0, 12.9, 13.3, 13.9, 15.8, 16.9, 20.2, 21.6, and 25.4.

According to some embodiments of the present disclosure, the Crystal Form VI, by Cu-K_{α} radiation, has an X-ray powder diffraction pattern substantially as shown in FIG. 6.

According to some embodiments of the present disclosure, the Crystal Form VI has a differential scanning calorimetric curve having an endothermic peak at 113.29±5°C.

According to some embodiments of the present disclosure, the Crystal Form VI has a thermogravimetric analysis curve having a weight loss of 0.34%±0.2% between room temperature and 120±5°C.

According to some embodiments of the present disclosure, the crystalline form of the compound represented by formula (B) is a Crystal Form VII which, by Cu-K_{α} radiation, has an X-ray powder diffraction pattern that comprises characteristic diffraction peaks at 2θ angles (±0.2°) of 4.3, 6.9, 13.2, 19.1, and 20.0.

According to some embodiments of the present disclosure, the Crystal Form VII, by Cu-K_{α} radiation, has an X-ray powder diffraction pattern that comprises characteristic diffraction peaks at 2θ angles (±0.2°) of 4.3, 6.9, 8.9, 13.2, 19.1, 20.0, and 21.7.

According to some embodiments of the present disclosure, the Crystal Form VII, by Cu-K_{α} radiation, has an X-ray powder diffraction pattern that comprises characteristic diffraction peaks at 2θ angles (±0.2°) of 4.3, 6.9, 8.9, 13.2, 15.1, 19.1, 20.0, 21.1, and 21.7.

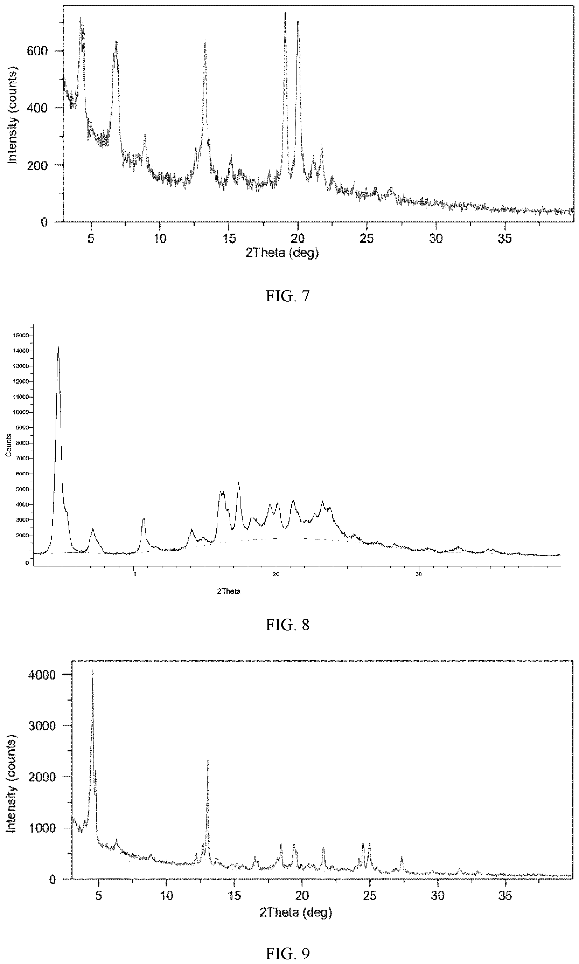

According to some embodiments of the present disclosure, the Crystal Form VII, by Cu-K_{α} radiation, has an X-ray powder diffraction pattern substantially as shown in FIG. 7.

According to some embodiments of the present disclosure, the Crystal Form VII has a differential scanning calorimetric curve having an endothermic peak at 197.3±5°C.

According to some embodiments of the present disclosure, the Crystal Form VII has a differential scanning calorimetric curve having endothermic peaks at 92.4±5°C and 197.3±5°C.

According to some embodiments of the present disclosure, the Crystal Form VII has a thermogravimetric analysis curve having a weight loss of 7.88%±0.2% between room temperature and 110±5°C.

According to some embodiments of the present disclosure, the compound represented by formula (A-1) is a compound represented by formula (C): 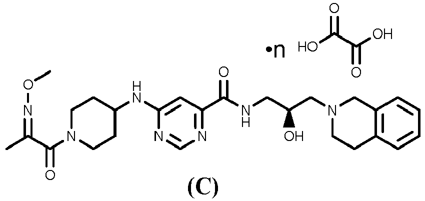 wherein n is selected from 0.5-2; preferably 1-1.5; further preferably 1, 1.1, 1.2, 1.3, 1.4 or 1.5; still further preferably 1 or 1.5.

According to some embodiments of the present disclosure, the compound represented by formula (C) is in a solid form.

According to some embodiments of the present disclosure, the solid form of the compound represented by formula (C), using KBr pellet method, has an infrared spectrum comprising characteristic peaks at the following positions (±4 cm⁻¹): 3320, 2937, 1615, 1526, 1456, 1368, 1047.

According to some embodiments of the present disclosure, the solid form of the compound represented by formula (C) is a crystalline form.

According to some embodiments of the present disclosure, the compound represented by formula (C) is a compound represented by formula (C-1) or formula (C-2):

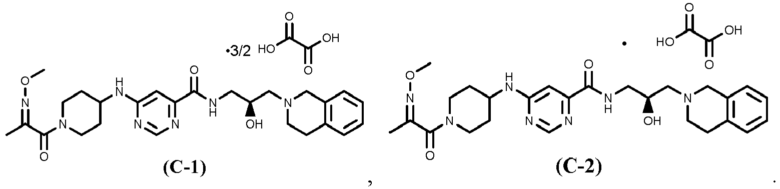

According to some embodiments of the present disclosure, the compound represented by formula (C-1) or the compound represented by formula (C-2) is in a solid form.

According to some embodiments of the present disclosure, the solid form of the compound represented by formula (C-1) or the compound represented by formula (C-2) is a crystalline form.

According to some embodiments of the present disclosure, the crystalline form of the compound represented by formula (C) is a Crystal Form I which, by Cu-K_{α} radiation, has an X-ray powder diffraction pattern that comprises characteristic diffraction peaks at 2θ angles (±0.2°) of 4.7, 7.1, 10.7, 17.4, and 21.2.

According to some embodiments of the present disclosure, the crystalline form of the compound represented by formula (C) is a Crystal Form I which, by Cu-K_{α} radiation, has an X-ray powder diffraction pattern that comprises characteristic diffraction peaks at 2θ angles (±0.2°) of 4.7, 7.1, 10.7, 16.1, 17.4, 20.1, and 21.2.

According to some embodiments of the present disclosure, the crystalline form of the compound represented by formula (C) is a Crystal Form I which, by Cu-K_{α} radiation, has an X-ray powder diffraction pattern that comprises characteristic diffraction peaks at 2θ angles (±0.2°) of 4.7, 7.1, 10.7, 16.1, 17.4, 19.6, 20.1, 21.2, and 23.2.

According to some embodiments of the present disclosure, the crystalline form of the compound represented by formula (C) is a Crystal Form I which, by Cu-K_{α} radiation, has an X-ray powder diffraction pattern substantially as shown in FIG. 8.

According to some embodiments of the present disclosure, the crystalline form of the compound represented by formula (C) is a Crystal Form II which, by Cu-K_{α} radiation, has an X-ray powder diffraction pattern that comprises characteristic diffraction peaks at 2θ angles (±0.2°) of 4.6, 13.0, and 21.6.

According to some embodiments of the present disclosure, the crystalline form of the compound represented by formula (C) is a Crystal Form II which, by Cu-K_{α} radiation, has an X-ray powder diffraction pattern that comprises characteristic diffraction peaks at 2θ angles (±0.2°) of 4.6, 13.0, 21.6, 24.5, and 25.0.

According to some embodiments of the present disclosure, the crystalline form of the compound represented by formula (C) is a Crystal Form II which, by Cu-K_{α} radiation, has an X-ray powder diffraction pattern that comprises characteristic diffraction peaks at 2θ angles (±0.2°) of 4.6, 4.8, 13.0, 18.4, 19.4, 21.6, 24.5, and 25.0.

According to some embodiments of the present disclosure, the crystalline form of the compound represented by formula (C) is a Crystal Form II which, by Cu-K_{α} radiation, has an X-ray powder diffraction pattern substantially as shown in FIG. 9.

According to some embodiments of the present disclosure, the crystalline form of the compound represented by formula (C) is a Crystal Form II which has a differential scanning calorimetric curve having an endothermic peak at 162.6±5°C.

According to some embodiments of the present disclosure, the crystalline form of the compound represented by formula (C) is a Crystal Form II which has a thermogravimetric analysis curve having a weight loss of 3.2%±0.2% between room temperature and 160±5°C.

According to some embodiments of the present disclosure, the crystalline form of the compound represented by formula (C) is a Crystal Form III which, by Cu-K_{α} radiation, has an X-ray powder diffraction pattern that comprises characteristic diffraction peaks at 2θ angles (±0.2°) of 4.6, 18.7, and 19.4.

According to some embodiments of the present disclosure, the crystalline form of the compound represented by formula (C) is a Crystal Form III which, by Cu-K_{α} radiation, has an X-ray powder diffraction pattern that comprises characteristic diffraction peaks at 2θ angles (±0.2°) of 4.6, 4.8, 14.0, 16.7, 18.7, 19.4, and 23.3.

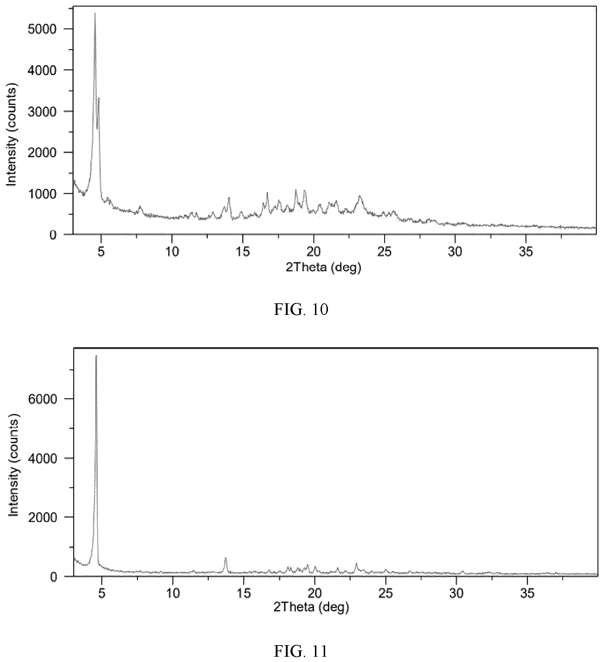

According to some embodiments of the present disclosure, the crystalline form of the compound represented by formula (C) is a Crystal Form III which, by Cu-K_{α} radiation, has an X-ray powder diffraction pattern substantially as shown in FIG. 10.

According to some embodiments of the present disclosure, the crystalline form of the compound represented by formula (C) is a Crystal Form III which has a differential scanning calorimetric curve having an endothermic peak at 204.9±5°C.

According to some embodiments of the present disclosure, the crystalline form of the compound represented by formula (C) is a Crystal Form III which has a differential scanning calorimetric curve having endothermic peaks at 139.9±5°C and 204.9±5°C.

According to some embodiments of the present disclosure, the crystalline form of the compound represented by formula (C) is a Crystal Form III which has a thermogravimetric analysis curve having a weight loss of 6.2%±0.2% between room temperature and 150±5°C.

According to some embodiments of the present disclosure, the crystalline form of the compound represented by formula (C) is a Crystal Form IV which, by Cu-K_{α} radiation, has an X-ray powder diffraction pattern that comprises characteristic diffraction peaks at 2θ angles (±0.2°) of 4.6, 13.7, 19.5, 20.0, and 22.9.

According to some embodiments of the present disclosure, the crystalline form of the compound represented by formula (C) is a Crystal Form IV which, by Cu-K_{α} radiation, has an X-ray powder diffraction pattern substantially as shown in FIG. 11.

According to some embodiments of the present disclosure, the crystalline form of the compound represented by formula (C) is a Crystal Form IV which, has a differential scanning calorimetric curve having an endothermic peak at 137.4±5°C.

According to some embodiments of the present disclosure, the crystalline form of the compound represented by formula (C) is a Crystal Form IV which has a thermogravimetric analysis curve having a weight loss of 4.26%±0.2% between room temperature and 150±5°C.

According to some embodiments of the present disclosure, the crystalline form of the compound represented by formula (C) is a Crystal Form V by Cu-K_{α} radiation, and the resulting single crystal is attributed to a tri-oblique crystal system, a P1 space group, with the following unit cell parameters: {a=5.55690 (10) Å, b=16.9102(2) Å, c=18.9473 (2) Å, *α*=99.1280(10)°, *β*=90.1780(10)°, *γ*=95.1340(10)°, *V*=1750.57 (4) Å³}.

In a second aspect, the present disclosure provides a crystalline composition, comprising one or more of the Crystal Form I, Crystal Form II, Crystal Form III, Crystal Form IV, Crystal Form V, Crystal Form VI and Crystal Form VII of the compound represented by formula (B).

According to some embodiments of the present disclosure, in the crystalline composition, the Crystal Form I, Crystal Form II, Crystal Form III, Crystal Form IV, Crystal Form V, Crystal Form VI or Crystal Form VII of the compound represented by formula (B) accounts for 50% or more, 60% or more, 70% or more, 80% or more, 90% or more or 95% or more by weight of the crystalline composition.

In a third aspect, the present disclosure provides a crystalline composition comprising one or more of the Crystal Form I, Crystal Form II, Crystal Form III, Crystal Form IV and Crystal Form V of the compound represented by Formula (C).

According to some embodiments of the present disclosure, in the crystalline composition, the Crystal Form I, Crystal Form II, Crystal Form III, Crystal Form IV or Crystal Form V of the compound represented by formula (C) accounts for 50% or more, 60% or more, 70% or more, 80% or more, 90% or more or 95% or more by weight of the crystalline composition.

In a fourth aspect, the present disclosure provides a pharmaceutical composition comprising a salt of a compound represented by formula (A), a compound represented by formula (A-1), a solid form of the compound represented by formula (A-1), a crystalline form of the compound represented by formula (A-1), a compound represented by formula (B), a solid form of the compound represented by formula (B), a crystalline form of the compound represented by formula (B), a compound represented by formula (B-1), a solid form of the compound represented by formula (B-1), a crystalline form of the compound represented by formula (B-1), a Crystal Form I of the compound represented by formula (B), a Crystal Form II of the compound represented by formula (B), a Crystal Form III of the compound represented by formula (B), a Crystal Form IV of the compound represented by formula (B), a Crystal Form V of the compound represented by formula (B), a Crystal Form VI of the compound represented by formula (B), a Crystal Form VII of the compound represented by formula (B), a compound represented by formula (C), a solid form of the compound represented by formula (C), a crystalline form of the compound represented by formula (C), a compound represented by formula (C-1), a compound represented by formula (C-2), a solid form of the compound represented by formula (C-1) or the compound represented by formula (C-2), a crystalline form of the compound represented by formula (C-1) or the compound represented by formula (C-2), a Crystal Form I of the compound represented by formula (C), a Crystal Form II of the compound represented by formula (C), a Crystal Form III of the compound represented by formula (C), a Crystal Form IV of the compound represented by formula (C), a Crystal Form V of the compound represented by formula (C), or the crystalline composition in the second aspect or the third aspect as described above.

According to some embodiments of the present disclosure, the pharmaceutical composition further comprises a pharmaceutically acceptable carrier.

In a fifth aspect, the present disclosure provides the salt of the compound represented by formula (A), the compound represented by formula (A-1), the solid form of the compound represented by formula (A-1), the crystalline form of the compound represented by formula (A-1), the compound represented by formula (B), the solid form of the compound represented by formula (B), the crystalline form of the compound represented by formula (B), the compound represented by formula (B-1), the solid form of the compound represented by formula (B-1), the crystalline form of the compound represented by formula (B-1), the Crystal Form I of the compound represented by formula (B), the Crystal Form II of the compound represented by formula (B), the Crystal Form III of the compound represented by formula (B), the Crystal Form IV of the compound represented by formula (B), the Crystal Form V of the compound represented by formula (B), the Crystal Form VI of the compound represented by formula (B), the Crystal Form VII of the compound represented by formula (B), the compound represented by formula (C), the solid form of the compound represented by formula (C), the crystalline form of the compound represented by formula (C), the compound represented by formula (C-1), the compound represented by formula (C-2), the solid form of the compound represented by formula (C-1) or the compound represented by formula (C-2), the crystalline form of the compound represented by formula (C-1) or the compound represented by formula (C-2), the Crystal Form I of the compound represented by formula (C), the Crystal Form II of the compound represented by formula (C), the Crystal Form III of the compound represented by formula (C), the Crystal Form IV of the compound represented by formula (C), or the Crystal Form V of the compound represented by formula (C) described in each of the above aspects, the crystalline composition in the second aspect or the third aspect as described above, or the pharmaceutical composition in the fourth aspect as described above, for use as a medicament, or the use thereof in the preparation of a medicament.

According to some embodiments of the present disclosure, the medicament is used for preventing and/or treating a cell proliferative disease; preferably, the cell proliferative disease is a tumor or cancer; more preferably, the tumor or cancer is a hematological neoplasm or a solid tumor; further preferably a malignant hematological neoplasm or an advanced solid tumor; further preferably a relapsed or refractory hematological neoplasm or an advanced malignant solid tumor.

According to some embodiments of the present disclosure, the medicament is used for preventing and/or treating diseases mediated at least partially by PRMT5.

In a sixth aspect, the present disclosure provides the salt of the compound represented by formula (A), the compound represented by formula (A-1), the solid form of the compound represented by formula (A-1), the crystalline form of the compound represented by formula (A-1), the compound represented by formula (B), the solid form of the compound represented by formula (B), the crystalline form of the compound represented by formula (B), the compound represented by formula (B-1), the solid form of the compound represented by formula (B-1), the crystalline form of the compound represented by formula (B-1), the Crystal Form I of the compound represented by formula (B), the Crystal Form II of the compound represented by formula (B), the Crystal Form III of the compound represented by formula (B), the Crystal Form IV of the compound represented by formula (B), the Crystal Form V of the compound represented by formula (B), the Crystal Form VI of the compound represented by formula (B), the Crystal Form VII of the compound represented by formula (B), the compound represented by formula (C), the solid form of the compound represented by formula (C), the crystalline form of the compound represented by formula (C), the compound represented by formula (C-1), the compound represented by formula (C-2), the solid form of the compound represented by formula (C-1) or the compound represented by formula (C-2), the crystalline form of the compound represented by formula (C-1) or the compound represented by formula (C-2), the Crystal Form I of the compound represented by formula (C), the Crystal Form II of the compound represented by formula (C), the Crystal Form III of the compound represented by formula (C), the Crystal Form IV of the compound represented by formula (C), or the Crystal Form V of the compound represented by formula (C) described in each of the above aspects, the crystalline composition in the second aspect or the third aspect as described above, or the pharmaceutical composition in the fourth aspect as described above, for use in the prevention and/or treatment of diseases mediated at least in part by PRMT5 or cell proliferative disorders.

In a seventh aspect, the present disclosure provides a method for preventing and/or treating diseases mediated at least in part by PRMT5 or cell proliferative disorders, comprising: administering a therapeutically effective amount of the salt of the compound represented by formula (A), the compound represented by formula (A-1), the solid form of the compound represented by formula (A-1), the crystalline form of the compound represented by formula (A-1), the compound represented by formula (B), the solid form of the compound represented by formula (B), the crystalline form of the compound represented by formula (B), the compound represented by formula (B-1), the solid form of the compound represented by formula (B-1), the crystalline form of the compound represented by formula (B-1), the Crystal Form I of the compound represented by formula (B), the Crystal Form II of the compound represented by formula (B), the Crystal Form III of the compound represented by formula (B), the Crystal Form IV of the compound represented by formula (B), the Crystal Form V of the compound represented by formula (B), the Crystal Form VI of the compound represented by formula (B), the Crystal Form VII of the compound represented by formula (B), the compound represented by formula (C), the solid form of the compound represented by formula (C), the crystalline form of the compound represented by formula (C), the compound represented by formula (C-1), the compound represented by formula (C-2), the solid form of the compound represented by formula (C-1) or the compound represented by formula (C-2), the crystalline form of the compound represented by formula (C-1) or the compound represented by formula (C-2), the Crystal Form I of the compound represented by formula (C), the Crystal Form II of the compound represented by formula (C), the Crystal Form III of the compound represented by formula (C), the Crystal Form IV of the compound represented by formula (C), the Crystal Form V of the compound represented by formula (C) described in each of the above aspects, the crystalline composition in the second aspect or the third aspect as described above, or the pharmaceutical composition in the fourth aspect as described above, to an subject in need thereof.

In some embodiments of the present disclosure, the above-mentioned disease according to the fifth aspect, the sixth aspect or the seventh aspect that is at least partially mediated by PRMT5 is a cell proliferative disease.

In some embodiments of the present disclosure, the above-mentioned cell proliferative disease according to the fifth aspect, the sixth aspect or the seventh aspect is a tumor or cancer; preferably, the tumor or cancer is a hematological neoplasm or a solid tumor; further preferably a malignant hematological neoplasm or an advanced solid tumor; still further preferably a relapsed or refractory hematologic neoplasm or an advanced malignant solid tumor.

In some embodiments of the present disclosure, the above-mentioned tumor or cancer according to the fifth aspect, the sixth aspect or the seventh aspect is selected from the group consisting of lung cancer, bone cancer, stomach cancer, pancreatic cancer, adenoid cystic carcinoma, skin cancer, head and neck cancer, uterine cancer, ovarian cancer, testicular cancer, fallopian tube cancer, endometrial carcinoma, cervical cancer, vaginal cancer, brain cancer, pituitary adenoma, melanoma, epidermoid carcinoma, and chronic and acute leukemia; preferably, the acute leukemia is acute myeloid leukemia (AML).

In an eighth aspect, the present disclosure provides a method for preparing a compound represented by formula (A-1), comprising: reacting the compound represented by formula (A) with an acid in a suitable solvent, and separating to obtain the compound represented by formula (A-1): 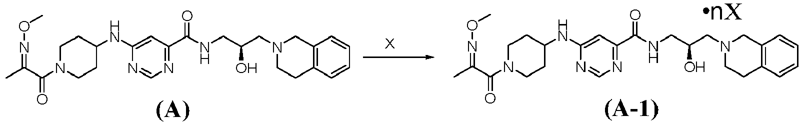 wherein X is an acid; preferably an inorganic acid or an organic acid; and n is 0.5-2.

According to some embodiments of the present disclosure, n is selected from 1-1.5; preferably 1, 1.1, 1.2, 1.3, 1.4 or 1.5; further preferably 1, 1.3, 1.4 or 1.5; still further preferably 1.

According to some embodiments of the present disclosure, X is an inorganic acid selected from sulfuric acid and phosphoric acid.

According to some embodiments of the present disclosure, X is an organic acid selected from malic acid, oxalic acid, succinic acid, tartaric acid, adipic acid, citric acid, gluconic acid, maleic acid, fumaric acid, lactic acid and gentisic acid; preferably malic acid, oxalic acid, succinic acid, L-tartaric acid, L-lactic acid, adipic acid, citric acid or gluconic acid; preferably malic acid, oxalic acid, succinic acid, L-tartaric acid, adipic acid, citric acid or gluconic acid; further preferably malic acid, oxalic acid, citric acid or gluconic acid; still further preferably malic acid or oxalic acid; still further preferably L-malic acid or oxalic acid.

According to the preparation method of the present disclosure, the molar ratio of the compound represented by formula (A) to the acid is 1-2:0.5-2, preferably 1:1-2, further preferably 1:1.1-2.

According to the preparation method of the present disclosure, the reaction temperature is 0-90°C, preferably 5-80°C, preferably 20-60°C, more preferably room temperature to 50°C.

According to the preparation method of the present disclosure, the reaction solvent is selected from one or a combination of two of alcohols, esters, nitriles, ketones, water, alkane-based solvents, ether-based solvents or heterocycloalkane-based solvents; preferably one or a combination of two of ROH, RCOOR₁, RCN, RCOR₁, water, ROR_{1,} RH or heterocycloalkane-based solvents, wherein R and R₁ are each independently selected from a C₁₋₆ linear or branched alkyl; preferably, R and R₁ are each independently selected from a C₁₋₄ linear or branched alkyl; preferably, the reaction solvent is selected from one or a combination of two of isopropanol, methanol, ethanol, ethyl acetate, acetone, butanone, acetonitrile, water, tetrahydrofuran, n-heptane and 2-methyltetrahydrofuran; when the reaction solvent is a mixed solvent composed of two solvents, the volume ratio of the two solvents is 1-20:20-1, preferably 1-19:19-1, preferably 1-10: 10-1.

According to the preparation method of the present disclosure, after the reaction is completed, optionally, the temperature is reduced to -15-15°C; the mixture is allowed to stand for crystallization for 0.5 h to 5 days, and the solid is separated and dried to obtain the compound represented by formula (A-1). Preferably, the crystallization temperature is 5°C, and the crystallization time is 1 h to 3 days.

According to the preparation method of the present disclosure, the separation step comprises separating the compound represented by formula (A-1) from the crystallization solution by a suitable method such as suction filtration, extraction filtration, filtration, centrifugation and the like.

According to the preparation method of the present disclosure, the drying method can be any suitable known method, preferably drying at room temperature, vacuum drying at room temperature or oven drying at 50°C. Specific drying conditions are, for example, a drying time of preferably 1 h to 5 days, more preferably 3 h to 3 days, and more preferably 3 h to 1 day. Regardless of the drying method, it is preferable that the residual amount of solvent in the obtained product satisfies the quality standard.

### Definition and Description

Unless otherwise specified, the following terms and phrases as used herein are intended to have the following meanings. A particular phrase or term should not be considered indefinite or unclear without a specific definition, but should be understood in the ordinary meaning.

The compound represented by formula (A) mentioned in the present disclosure may be present in specific stereoisomer forms, including *cis-* and *trans*-isomers, (R)-and (S)-enantiomers, racemic mixtures thereof and the like, preferably a *trans*-isomer.

The "solid form of the salt of the compound represented by formula (A) or the compound represented by formula (A-1)", "solid form of the compound represented by formula (B)" or "solid form of the compound represented by formula (C)" and the like mentioned in the present disclosure refer to the compound represented by formula (A-1) in a solid form, the compound represented by formula (B) in a solid form, the compound represented by formula (C) in a solid form, and the like, including a crystalline form, an amorphous form and the like of the compound represented by formula (A-1), the compound represented by formula (B), the compound represented by formula (C) or the compound represented by formula (C-2).

The "crystalline form of the salt of the compound represented by formula (A) or the compound represented by formula (A-1)", "crystalline form of the compound represented by formula (B)" or "crystalline form of the compound represented by formula (C)" and the like mentioned in the present disclosure refer to the compound represented by formula (A-1), the compound represented by formula (B), the compound represented by formula (C) and the like in a crystalline state, including an anhydrous and solvent-free form, a hydrate form, a solvate form and an eutectic form, of the compound represented by formula (A-1), the compound represented by formula (B) or the compound represented by formula (C).

The term "solvate" refers to a conjugate formed from solvent molecules and the salt of the compound represented by formula (A), the compound represented by formula (A-1), the compound represented by formula (B), the compound represented by formula (C) and the like of the present disclosure, in a stoichiometric or non-stoichiometric ratio, including a conjugate containing both water molecules and molecules of one or more other solvents, and a conjugate containing molecules of one or more other solvents alone.

The term "hydrate" refers to a conjugate formed from water molecules and the salt of the compound represented by formula (A), the compound represented by formula (A-1), the compound represented by formula (B), the compound represented by formula (C) and the like of the present disclosure, in a stoichiometric or non-stoichiometric ratio.

An "anhydrous and solvent-free form" means that no water molecule or solvent molecules are included, or water molecules or solvent molecules co-exist with the salt of the compound represented by formula (A), the compound represented by formula (A-1), the compound represented by formula (B), the compound represented by formula (C) and the like in a manner of non-intermolecular binding, for example, by adsorption.

The term "crystalline composition" refers to a solid form comprising one or more of the specific crystal forms of the compound represented by formula (A), the salt of the compound represented by formula (A-1), the compound represented by formula (B), the compound represented by formula (C) and the like mentioned in the present disclosure. For example, in one embodiment according to the present disclosure, the crystalline composition comprises one, two or more of the Crystal Form I, Crystal Form II, Crystal Form III, Crystal Form IV, Crystal Form V, Crystal Form VI and Crystal Form VII of the compound represented by formula (B) mentioned in the present disclosure. Moreover, in addition to the crystal forms of the present disclosure, the crystalline composition may optionally comprise other crystalline forms, other crystal forms or other amorphous forms of the compounds (the compound represented by formula (A-1), the compound represented by formula (B), the compound represented by formula (B-1), the compound represented by formula (C), the compound represented by formula (C-1) or the compound represented by formula (C-2)), or impurities other than these substances. It should be understood by those skilled in the art that the sum of the contents of the ingredients in the crystalline composition should be 100%.

"Room temperature" is a room temperature in a conventional sense in the art, and is generally 10-30°C, preferably 25°C±5°C.

In the context of the present disclosure, the 2θ values in the X-ray powder diffraction patterns are in terms of angles (°).

In an X-ray powder diffraction pattern, the term "substantially" or "substantially as shown in FIG." refers to a substantially pure crystal form in which at least 50%, or at least 60%, or at least 70%, or at least 80%, or at least 90%, or at least 95%, or at least 96%, or at least 97%, or at least 98%, or at least 99% of the peaks in its powder X-ray diffraction pattern appear in the provided pattern. Further, when the content of a certain crystal form in the product gradually decreases, some diffraction peaks attributed to the crystal form in the X-ray powder diffraction pattern may be reduced due to factors of detection sensitivity of the instrument. Furthermore, there may be slight errors in the position of the peaks for any given crystal form, which is also well known in the field of crystallography. For example, when analyzing a sample, due to changes in temperature, sample displacement or instrument calibration, etc., the position of the peaks may shift, and the error of measurement for a 2θ value is usually about ±0.2°. Thus, such an error should be taken into consideration when determining the structure of each crystal form, and the term "substantially" or "substantially as shown in FIG." is intended to encompass such a difference in the position of a diffraction peak.

In a DSC pattern or TGA pattern, the term "substantially" or "substantially as shown in FIG." means that for the same crystal form of the same compound, in a continuous analysis, the error of the thermal transition onset temperature, endothermic peak temperature, exothermic peak temperature, melting point, weight loss onset temperature, or weight loss endpoint temperature are typically within about 5°C, usually within about 3°C. When a compound is described to have a given thermal transition onset temperature, endothermic peak temperature, exothermic peak temperature, melting point, weight loss onset temperature, weight loss endpoint temperature, or the like, it refers to the temperature ±5°C.

The term "cell proliferative disease" as used herein refers to a condition in which a population of cells grow at a rate lower or higher than that predicted under certain physiological states and conditions.

The term "tumor" includes benign tumors, malignant tumors, and borderline tumors, where the malignant tumors are also collectively referred to as cancer.

The term "prevention" as used herein means that when used in reference to a disease or condition (*e.g.,* cancer), the compound or drug (*e.g.,* a combination product claimed herein) is capable of reducing the frequency or delaying the onset of symptoms of a medical condition in a subject, as compared to a subject to whom the compound or drug is not administered.

The term "treatment" as used herein refers to the alleviation of, relief from, or amelioration of symptoms of a disease or condition, amelioration of potential metabolism-induced symptoms, suppression of a disease or symptoms, *e.g*., prevented progression of a disease or condition, relief from a disease or condition, induced regression of a disease or condition, relief from pathological states induced by a disease or condition, or prevention of symptoms of a disease or condition.

The term "pharmaceutically acceptable carrier" or "pharmaceutically acceptable excipient" refers to those carriers that neither significantly irritate to an organism, nor impair the biological activity and performances of an active compound.

The intermediate compounds in the present disclosure can be prepared by a variety of synthetic methods well known to those skilled in the art, including specific embodiments as listed below, embodiments formed by their combination with other chemical synthesis methods, and equivalent alternatives well known to those skilled in the art. Preferred embodiments include, but are not limited to, the examples of the present application.

The chemical reactions of the specific embodiments of the present disclosure are carried out in suitable solvents which are adapted to the chemical changes of the present disclosure and the required reagents or materials thereof. In order to obtain the compounds of the present disclosure, it is sometimes necessary for those skilled in the art to modify or select synthesis steps or reaction schemes on the basis of the existing embodiments.

The present disclosure will be described in detail by way of examples, which are not intended to be any limitation to the present disclosure.

All solvents used in the present disclosure are commercially available, and can be used without further purification.

The "stoichiometric ratio" in the present disclosure refers to a molar ratio, i.e., a molar ratio of a compound in a free form to an acid.

The results of *in vitro* enzymatic experiments show: as compared to a control compound, the compound represented by formula (A) has better inhibitory activities against PRMT5 and MV4-11 cells.

The results of *in vivo* anti-tumor experiment show: as compared to the control compound, the compound represented by formula (A) has a significant anti-tumor activity, and have significantly better inhibitory effects on tumor weight and tumor volume.

The results of *in vivo* pharmacokinetic experiment show: the compound represented by formula (A) has better performance of oral administration, high absorption rate, and good absorption performance.

On the basis of one or more effects in the above (1), (2) or (3), the salt of the compound represented by formula (A) has valuable application prospects.

The salt of the compound represented by formula (A) or the compound represented by formula (A-1) is obtained for the first time.

The solid form of the salt of the compound represented by formula (A) or the compound represented by formula (A-1), and the crystalline form of the salt of the compound represented by formula (A) or the compound represented by formula (A-1) are obtained for the first time, which are convenient to separate, transfer and weigh.

The salt of the compound represented by formula (A) or the compound represented by formula (A-1) has a good dissolution effect.

The solid form of the compound represented by formula (B), the compound represented by formula (B-1), the compound represented by formula (C), and the compound represented by formula (C-1) or the compound represented by formula (C-2) are obtained for the first time, which has good morphological character and is convenient to separate, transfer and weigh.

The solid form of the compound represented by formula (B), the compound represented by formula (B-1), the compound represented by formula (C), the compound represented by formula (C-1) or the compound represented by formula (C-2) can be prepared and separated in a high purity (greater than 95%) and/or yield (greater than 80%).

The crystalline form of the compound represented by formula (B), the crystalline form of the compound represented by formula (B-1), the crystalline form of the compound represented by formula (C), and the crystalline form of the compound represented by formula (C-1) or the compound represented by formula (C-2) have good crystallization.

The crystalline form of the compound represented by formula (B) and specific crystal forms thereof and the crystalline form of the compound represented by formula (C) and specific crystal forms thereof are easy to purify and separate (e.g., filter), and the preparation is simple and convenient.

It is preferable that the crystal forms have good physical stability and chemical stability, and have good prospects in pharmaceutical application.

### Description of the accompanying drawings

FIG. 1: X-ray powder diffraction pattern of the Crystal Form I of the compound represented by formula (B) of Example 1.
FIG. 2: X-ray powder diffraction pattern of the Crystal Form II of the compound represented by formula (B) of Example 2.
FIG. 3: X-ray powder diffraction pattern of the Crystal Form III of the compound represented by formula (B) of Example 3.
FIG. 4: X-ray powder diffraction pattern of the Crystal Form IV of the compound represented by formula (B) of Example 4.
FIG. 5: X-ray powder diffraction pattern of the Crystal Form V of the compound represented by formula (B) of Example 5.
FIG. 6: X-ray powder diffraction pattern of the Crystal Form VI of the compound represented by formula (B) of Example 6.
FIG. 7: X-ray powder diffraction pattern of the Crystal Form VII of the compound represented by formula (B) of Example 7.
FIG. 8: X-ray powder diffraction pattern of the Crystal Form I of the compound represented by formula (C) of Example 8.
FIG. 9: X-ray powder diffraction pattern of the Crystal Form II of the compound represented by formula (C) of Example 9.
FIG. 10: X-ray powder diffraction pattern of the Crystal Form III of the compound represented by formula (C) of Example 10.
FIG. 11: X-ray powder diffraction pattern of the Crystal Form IV of the compound represented by formula (C) of Example 11.

### Detailed Description of the Invention

### 1. X-ray powder diffractometer, XRPD

| | | | |
|---|---|---|---|
| Instrument Model | Panalytical X'Pert³ | Bruker D8 advance | D2 PHASER |
| Test sample | Examples 7, 9-11, 13-14 | Examples 1-6 | Example 8 |
| Sample amount | 1-10 mg | 5-20 mg | 100-200 mg |
| X-ray generator | Cu, Kα, (Kα1: λ=1.540598 Å; Kα2: λ=1.54426 Å; intensity ratio Kα2/ Kα1: 0.50) | Cu, Kα, (λ=1.5418 Å) | Cu, Kα, (λ=1.54060 Å) |
| Light tube voltage and current | 45 kV, 40 mA | 40 kV, 40 mA | 30 kV, 10 mA |
| Scan range (2θ) | 3°-40° | 3°-40° | 3°-40° |
| Scan step | 0.0263 | 0.02° | 0.02° |

### 2. Differential Scanning Calorimeter, DSC

| | | |
|---|---|---|
| Instrument Model | TA Discovery 2500 | TA Discovery DSC 250 |
| Test sample | Examples 7, 9-11, 13-14 | Examples 1-6 |
| Sample amount | 1-10 mg | 2-3 mg |
| Sample disc | Aluminum disc, covered | Aluminum disc, covered and perforated |
| Temperature range | 25-280°C | 25-250 |
| Heating rate | 10°C/min | 10°C/min |
| Protective gas | Nitrogen | Nitrogen |

### 3. Thermal Gravimetric Analyzer, TGA

| | | |
|---|---|---|
| Instrument Model | TA Discovery 5500 | TA Discovery TGA 55 |
| Test sample | Examples 7, 9-11, 13-14 | Examples 1-6 |
| Sample amount | 1-10 mg | 2-3 mg |
| Sample disc | Aluminum disc, open | Aluminum disc |
| Temperature range | Room temperature to 350°C | Room temperature to 300°C |
| Heating rate | 10°C/min | 10°C/min |
| Protective gas | Nitrogen | Nitrogen |

### 4. Nuclear Magnetic Resonance Spectroscopy (NMR)

Instrument model: Bruker 400M nuclear magnetic resonance spectrometer (Bruker, GER) Content and test solvent: ¹H-NMR, the test solvent was DMSO-d6.

### 5. Infrared Spectroscopy (IR)

### Detection instrument: Perkinelmer Spectrum 100 FT-IR infrared spectrum analyzer

Test method: 3 mg of a sample was weighed, diluted and pelleted with KBr, and measured at room temperature with the following parameters: detection range: 4000-400 cm⁻¹ wave number, resolution: 4 cm⁻¹.

### 6. Ion Chromatography (IC)

### Detection instrument: ThermoFisher ICS-1100 ion chromatograph

| | |
|---|---|
| Chromatographic column | Ionpac^{™} AS18A RFICTM 4×250mm Analytical |
| Injection volume | 25µL |
| Flow rate | 1.0 mL/min |
| Sample cell temperature/column temperature | 35°C/35°C |
| Current | 80 mA |

### 7. High Performance Liquid Chromatography (HPLC)

### Detection instrument: Agilent 1260

| | |
|---|---|
| Chromatographic column | Column filled with octadecylsilane-bonded silica gel (CAPCELL PAK C18 MGII, 4.6*250mm, 5 µm) |
| Detection wavelength | 205nm |
| Injection volume | 20µL |
| Flow rate | 0.7 mL/min |
| Column temperature | 45°C |
| Current | 80 mA |

### 8. Single crystal diffraction

| | |
|---|---|
| Instrument Model | Rigaku XtaLAB Synergy-R type single crystal diffractometer |
| X-ray light source | Mico-Max007HF Cu mode (Cu/Kα: 1.54184 Å) |
| Detector | Hypix6000HE Detector |
| Temperature | 120K |
| Analysis software | OLEX2 |

### 9. Solubility determination

FaSSIF: human fasted state simulated intestinal fluid; FeSSIF: human fed state simulated intestinal fluid; SGF: human fasted state simulated gastric fluid.

Test method: a sample to be tested was added into water or a biological solvent medium (4 mL); rotated and mixed (about 25 rpm) on a rotary incubator at 37°C; about 0.8 mL of the solution or suspension was palced into a centrifuge tube at a sampling point (in the present disclosure, the sampling point was 24 h), and separated by centrifugation; the supernatant was filtrated through a filter membrane, and the filtrate was tested for solubility.

In order to better understand the contents of the present disclosure, further description will be given below with reference to specific examples, but the specific examples are not intended to limit the contents of the present disclosure. Test methods for which specific conditions are not specified in the following preparation examples, examples, test examples or experimental examples are selected according to conventional methods and conditions or according to instructions of commercial products.

### Preparation Example 1: preparation of the compound of formula (A)

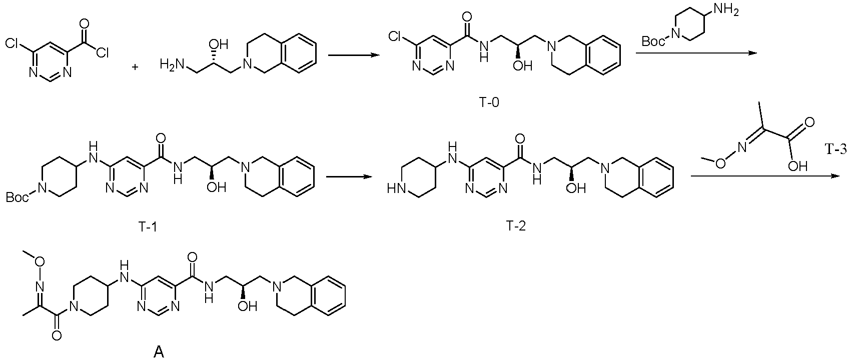

Preparation of intermediate T-0: In a 100 mL reaction flask, 6-chloropyrimidine-4-carbonyl chloride (0.63 g, 3.56 mmol) was dissolved in dichloromethane (10 mL), and at 0°C, triethylamine (0.72 g, 7.12 mmol) was added. Thereafter, (*S*)-1-amino-3 -(3,4-dihydroisoquinolin-2(*1H*)-yl)propan-2-ol (0.66 g, 3.20 mmol) was added. The reaction solution was stirred at 25°C for 2 h. After the reaction was completed (monitored by TLC), the reaction solution was diluted with H₂O (5 mL), and extracted with dichloromethane (15 mL×2). The combined organic phase was washed with saturated saline (10 mL), dried over Na₂SO₄, filtered, and concentrated under reduced pressure to afford a residue. The residue was purified by column chromatography (DCM:MeOH=10:1, v/v, the same below) to give an intermediate compound T-0 (yellow oil, 0.68 g, 61.28%).

Preparation of intermediate T-1: (*S*)-6-chloro-*N*-(3-(3,4-dihydroisoquinolin-2(1*H*)-yl)-2-hydroxypropyl)pyrimidine-4-carboxamide (T-0, 0.28 g, 0.81 mmol) was dissolved in isopropanol (20 mL), and triethylamine (0.25 g, 2.47 mmol) and tert-butyl 4-aminopiperidine-1-carboxylate (0.24 g, 1.2 mmol) were added in sequence. The reaction solution was warmed up to 85°C and reacted for 8 h. After the reaction was completed (monitored by TLC), the reaction solution was concentrated under reduced pressure and purified by column chromatography (DCM:MeOH=30:1) to give an intermediate compound T-1 (0.36g, 87.32%).

Preparation of intermediate T-2: Tert-butyl (*S*)-4-((6-(3-(3,4-dihydroisoquinolin-2(1*H*)-yl)-2-hydroxypropyl)carbamoyl)pyrimidin-4-yl)amino)piperidin-1-yl)carbamate (T-1, 0.36 g, 0.71 mmol) was dissolved in dichloromethane (20 mL), and trifluoroacetic acid (1 mL) was added and reacted at room temperature for 1 h. The reaction solution was concentrated to dryness under reduced pressure to give an intermediate compound T-2 (0.25 g, 86.38%). LC-MS: m/z 411.54 [M+H]⁺.

Preparation of intermediate T-3: 2-oxopropanoic acid (0.69 g, 7.84 mmol, 1eq) was dissolved in a mixed solvent of tetrahydrofuran (20 mL) and water (100 mL), and KOH (0.48 g, 8.62 mmol, 1.1 eq) was added, and stirred until the reaction solution was clear. Thereafter, under the ice bath condition, methoxyamine hydrochloride (1.31 g, 15.68 mmol, 2eq) was added in batches, and reacted for 2 h. The reaction solution was filtered to give an intermediate compound T-3 (0.91 g, 77.78%). ¹H NMR (600 MHz, DMSO-*d*₆): δ 1.920 (s, 3H), 3.953 (s, 3H), 12.985 (s, 1H).

Preparation of compound A: Intermediate T-2 (0.25 g, 0.61 mmol) was dissolved in DMF (20 mL), and TEA (3 mL, the volume could be adjusted as long as the pH value of the system was more than 10), intermediate T-3 (0.085 g, 0.73 mmol), and HATU (0.30 g, 0.789 mmol) were added in sequence and reacted at room temperature for 2 h. After the reaction was complete monitored by TLC, the reaction solution was added with water and ethyl acetate and subjected to liquid-liquid separation. The organic phase was washed with water and saturated saline respectively, each for once, and then concentrated. The residue was purified by column chromatography (dichloromethane:methanol=30:1) to give a target compound (0.22 g, 70.75%). LC-MS: m/z 510.34 [M+H]⁺. ¹H NMR (600 MHz, CD₃OD): δ 1.459-1.533 (m, 2H), 1.996 (s, 3H), 2.036-2.063 (m, 2H), 2.645-2.655 (m, 2H), 2.814-2.848 (m, 2H), 2.907-2.926 (m, 2H), 3.014 (m, 1H), 3.261-3.303 (m, 1H), 3.466-3.550 (m, 2H), 3.710 (s, 2H), 3.909 (s, 3H), 4.018-4.074 (m, 2H), 4.210 (s, 1H), 4.399-4.420 (d, 1H), 6.987-7.108 (m, 5H), 8.238(s, 1H).

### Example 1: Preparation of the compound of formula (B)

A sample of Preparation Example 1 (0.5 g) was weighed, dissolved in isopropanol (5mL) and added with L-malic acid (2 eq), stirred at room temperature for about 10 h, filtered, and dried at 50°C±5°C for 8 h, to obtain a solid with a yield of 92.5%. Upon detection, it was confirmed that a salt was formed with a base/acid ratio of 1: 1. IR (KBr, cm⁻¹): 3301.35, 2939.94, 1610.73, 1528.35, 1455.99, 1367.93, 1045.03.

A sample was subjected to X-ray powder diffraction, and was shown to be a crystalline solid (Crystal Form I) and well crystallized. The spectrum is shown in FIG. 1 and its XRPD diffraction peak data are shown in Table 1. A sample was taken for DSC-TGA testing. The DSC pattern showed endothermic peaks at 102.15°C and 113°C, and the TGA pattern showed that the sample had a weight loss of 8.6522% between room temperature and 110°C.

**Table 1. XRPD diffraction peak data of the sample obtained in Example 1**

| Peak position (2θ)° | Relative intensity, % | Peak position (2θ)° | Relative intensity, % | Peak position (2θ)° | Relative intensity, % |
|---|---|---|---|---|---|
| 4.205 | 100.0 | 13.329 | 10.3 | 20.111 | 6.1 |
| 6.534 | 13.2 | 18.277 | 6.5 | 21.986 | 7.8 |
| 8.532 | 4.7 | 19.067 | 17.8 | 22.399 | 5.1 |

| | | | | | |
|---|---|---|---|---|---|
| Note: peaks with relative peak intensity > 4.0% are listed in the table. | | | | | |

### Example 2: Preparation of Crystal Form II of the compound of formula (B)

The sample obtained in Example 1 (30 mg) was weighed and added into tetrahydrofuran (1.1 mL) to prepare a solution, into which n-heptane (0.4 mL) was added dropwise, and stirred at room temperature for 7 days. It was then filtered and dried under vacuum at 50°C for 2 h to obtain a solid. A sample was subjected to X-ray powder diffraction, and it was shown to be a crystalline solid (Crystal Form II) and well crystallized. The spectrum is shown in FIG. 2 and its XRPD diffraction peak data are shown in Table 2. A sample was taken for DSC-TGA testing. The DSC pattern showed an endothermic peak at 89.8°C and 103.58°C, respectively, and the TGA pattern showed that the sample had a weight loss of 2.6358% between room temperature and 80°C.

**Table 2. XRPD diffraction peak data of Crystal Form II of Example2**

| Peak position (2θ)° | Relative intensity, % | Peak position (2θ)° | Relative intensity, % | Peak position (2θ)° | Relative intensity, % |
|---|---|---|---|---|---|
| 4.924 | 69.1 | 18.674 | 100.0 | 23.294 | 24.7 |
| 6.619 | 81.0 | 19.815 | 79.3 | 24.012 | 18.9 |
| 12.296 | 22.7 | 20.281 | 41.6 | 24.384 | 18.4 |
| 13.228 | 87.2 | 21.044 | 13.7 | 26.532 | 34.8 |
| 13.394 | 75.3 | 22.100 | 45.1 | 28.798 | 12.2 |
| 16.487 | 12.9 | 23.285 | 25.8 | | |

| | | | | | |
|---|---|---|---|---|---|
| Note: peaks with relative peak intensity > 12.0% are listed in the table. | | | | | |

### Example 3: Preparation of Crystal Form III of the compound of formula (B)

A sample of Example 1 (about 50 mg) was weighed in a sample flask, acetonitrile (1.5 mL) was added, stirred at room temperature for 7 days, and filtered to obtain a solid. A sample was subjected to X-ray powder diffraction, and it was shown to be a crystalline solid (Crystal Form III) and well crystallized. The spectrum is shown in FIG. 3 and its XRPD diffraction peak data are shown in Table 3. A sample was taken for DSC-TGA testing. The DSC pattern showed endothermic peaks at 70.82°C and 117.93°C, and the TGA pattern showed that the sample had a weight loss of 3.0001% between room temperature and 75°C.

**Table 3. XRPD diffraction peak data of Crystal Form III of Example 3**

| Peak position (2θ)° | Relative intensity, % | Peak position (2θ)° | Relative intensity, % | Peak position (2θ)° | Relative intensity, % |
|---|---|---|---|---|---|
| 4.332 | 100.0 | 12.846 | 24.9 | 15.888 | 12.1 |
| 6.919 | 55.8 | 13.310 | 39.4 | 20.250 | 65.5 |
| 8.800 | 28.1 | 13.961 | 11.0 | 21.194 | 23.4 |

| | | | | | |
|---|---|---|---|---|---|
| Note: peaks with relative peak intensity > 10.0% are listed in the table. | | | | | |

### Example 4: Preparation of Crystal Form IV of the compound of formula (B)

A sample of Example 1 (about 50 mg) was weighed in a sample flask, a mixed solution of methanol/methyl tert-butyl ether (1.5 mL, v/v=1/3) was added, stirred at room temperature for 7 days, and filtered to obtain a solid. A sample was subjected to X-ray powder diffraction, and it was shown to be a crystalline solid (Crystal Form IV) and well crystallized. The spectrum is shown in FIG. 4 and its XRPD diffraction peak data are shown in Table 4. A sample was taken for DSC-TGA testing. The DSC pattern showed two endothermic peaks at 77.91°C and 113.27°C, and the TGA pattern showed that the sample had a weight loss of 2.6271% between room temperature and 60°C.

**Table 4. XRPD diffraction peak data of Crystal Form IV of Example 4**

| Peak position (2θ)° | Relative intensity, % | Peak position (2θ)° | Relative intensity, % | Peak position (2θ)° | Relative intensity, % |
|---|---|---|---|---|---|
| 4.446 | 100.0 | 13.870 | 27.3 | 18.512 | 12.0 |
| 7.598 | 28.6 | 15.194 | 11.9 | 20.630 | 39.3 |
| 8.890 | 27.4 | 17.115 | 11.1 | 21.340 | 8.7 |
| 12.143 | 12.0 | 17.799 | 16.6 | | |

| | | | | | |
|---|---|---|---|---|---|
| Note: peaks with relative peak intensity >8% are listed in the table. | | | | | |

### Example 5: Preparation of Crystal Form V of the compound of formula (B)

A sample of Example 1 (about 50 mg) was weighed in a sample flask, acetone (1.5 mL) was added, stirred at room temperature for 7 days, and filtered to obtain a solid. A sample was subjected to X-ray powder diffraction, and it was shown to be a crystalline solid (Crystal Form V) and well crystallized. The spectrum is shown in FIG. 5 and its XRPD diffraction peak data are shown in Table 5. A sample was taken for DSC-TGA testing. The DSC pattern showed two endothermic peaks at 68.38°C and 104.69°C, and the TGA pattern showed that the sample had a weight loss of 3.6041% between room temperature and 75°C.

**Table 5. XRPD diffraction peak data of Crystal Form V of Example 5**

| Peak position (2θ)° | Relative intensity, % | Peak position (2θ)° | Relative intensity, % | Peak position (2θ)° | Relative intensity, % |
|---|---|---|---|---|---|
| 4.971 | 89.9 | 16.452 | 11.9 | 22.789 | 15.9 |
| 6.573 | 30.4 | 16.700 | 20.2 | 23.233 | 26.9 |
| 6.788 | 46.4 | 18.645 | 100.0 | 24.259 | 26.1 |
| 12.418 | 17.7 | 19.090 | 42.2 | 24.962 | 22.5 |
| 12.796 | 24.3 | 19.592 | 62.5 | 25.708 | 15.2 |
| 13.070 | 36.0 | 20.200 | 46.1 | 28.640 | 15.4 |
| 13.646 | 60.7 | 20.407 | 19.5 | | |
| 14.350 | 16.2 | 21.763 | 15.5 | | |

| | | | | | |
|---|---|---|---|---|---|
| Note: peaks with relative peak intensity >10.0% are listed in the table. | | | | | |

### Example 6: Preparation of Crystal Form VI of the compound of formula (B)

An appropriate amount of Crystal Form IV obtained in Example 4 was weighed and dried under vacuum at 50°C for 2 days to obtain a solid. A sample was subjected to X-ray powder diffraction, and it was shown to be a crystalline solid (Crystal Form VI) and well crystallized. The spectrum is shown in FIG. 6 and its XRPD diffraction peak data are shown in Table 6. A sample was taken for DSC-TGA testing. The DSC pattern showed an endothermic peak at 113.29°C, and the TGA pattern showed that the sample had a weight loss of 0.34% between room temperature and 120°C.

**Table 6. XRPD diffraction peak data of Crystal Form VI of Example 6**

| Peak position (2θ)° | Relative intensity, % | Peak position (2θ)° | Relative intensity, % | Peak position (2θ)° | Relative intensity, % |
|---|---|---|---|---|---|
| 4.520 | 95.3 | 13.284 | 56.2 | 19.326 | 11.3 |
| 6.956 | 100.0 | 13.904 | 21.8 | 20.157 | 79.8 |
| 9.026 | 85.3 | 15.845 | 27.2 | 21.613 | 33.9 |
| 12.921 | 74.5 | 16.931 | 13.3 | 25.367 | 11.7 |

| | | | | | |
|---|---|---|---|---|---|
| Note: peaks with relative peak intensity >10.0% are listed in the table. | | | | | |

### Example 7: Preparation of Crystal Form VII of the compound of formula (B)

A sample (501.3 mg) obtained in Preparation Example 1 was weighed in a 20 mL reaction flask, and ethanol (12 mL) was added to prepare a solution. L-malic acid (131.6 mg) was added, and magnetically stirred at room temperature for 8 days. The solid was centrifuged and dried under vacuum at room temperature for 1 day, with a purity of 98.22%. Upon NMR detection, it was confirmed that a salt was formed with a base/acid ratio of 1:1.5.

A sample was subjected to X-ray powder diffraction, and it was shown to be a crystalline solid (Crystal Form VII) and well crystallized. The spectrum is shown in FIG. 7 and its XRPD diffraction peak data are shown in Table 7. A sample was taken for DSC-TGA testing. The DSC pattern showed endothermic peaks at 92.4°C and 197.3°C, and the TGA pattern showed that the sample had a weight loss of 7.88% between room temperature and 110°C.

**Table 7. XRPD diffraction peak data of Crystal Form VII of Example 7**

| Peak position (2θ)° | Relative intensity, % | Peak position (2θ)° | Relative intensity, % | Peak position (2θ)° | Relative intensity, % |
|---|---|---|---|---|---|
| 4.22 | 90.01 | 8.92 | 24.47 | 19.05 | 100.00 |
| 4.44 | 86.27 | 12.56 | 19.47 | 19.98 | 94.97 |
| 6.58 | 66.12 | 13.24 | 80.99 | 21.09 | 19.27 |
| 6.91 | 67.56 | 15.12 | 15.06 | 21.70 | 25.80 |

| | | | | | |
|---|---|---|---|---|---|
| Note: peaks with relative peak intensity >15.0% are listed in the table. | | | | | |

### Example 8: Preparation of Crystal Form I of the compound of formula (C)

A sample of Preparation Example 1 (0.5 g) was weighed, dissolved in isopropanol (5 mL) and added with oxalic acid (2 eq), stirred at room temperature for about 10 h, filtered, and dried at 50°C±5°C for 6h, to obtain a solid with a yield of 90%. Upon detection, it was confirmed that a salt was formed. IR (KBr, cm⁻¹): 3319.96, 2936.96, 1615.04, 1526.12, 1455.78, 1368.16, 1046.84.

A sample was subjected to X-ray powder diffraction, and it was shown to be a crystalline solid (Crystal Form I) and well crystallized. The spectrum is shown in FIG. 8 and its XRPD diffraction peak data are shown in Table 8.

**Table 8. XRPD diffraction peak data of Crystal Form I of Example 8**

| Peak position (2θ)° | Relative intensity, % | Peak position (2θ)° | Relative intensity, % | Peak position (2θ)° | Relative intensity, % |
|---|---|---|---|---|---|
| 4.734 | 100.0 | 16.327 | 27.7 | 21.213 | 20.5 |
| 5.323 | 19.9 | 16.744 | 17.3 | 22.708 | 13.9 |
| 7.136 | 12.4 | 17.373 | 32.4 | 23.244 | 20.8 |
| 10.714 | 18.1 | 18.365 | 12.9 | 23.696 | 17.6 |
| 14.082 | 9.9 | 19.569 | 18.3 | | |
| 16.128 | 26.9 | 20.122 | 20.0 | | |

| | | | | | |
|---|---|---|---|---|---|
| Note: peaks with relative peak intensity >9.0% are listed in the table. | | | | | |

### Example 9: Preparation of Crystal Form II of the compound of formula (C)

Oxalic acid dihydrate (6.4 mg) was weighed and added into a vial, into which an ethanol solution (40 mg/mL, 0.5 mL) of a sample of Preparation Example 1 was added, and magnetically stirred at room temperature for 3 days. The solid was separated by centrifugation and dried under vacuum at room temperature for 1 day, with a purity of 98.56%. Upon detection, it was confirmed that a salt was formed with a base/acid ratio of 1:1.5.

A sample was subjected to X-ray powder diffraction, and it was shown to be a crystalline solid (Crystal Form II) and well crystallized. The spectrum is shown in FIG. 9 and its XRPD diffraction peak data are shown in Table 9. A sample was taken for DSC-TGA testing. The DSC pattern showed an endothermic peak at 162.6°C, and the TGA pattern showed that the sample had a weight loss of 3.20% between room temperature and 160°C.

**Table 9. XRPD diffraction peak data of Crystal Form II of Example 9**

| Peak position (2θ)° | Relative intensity, % | Peak position (2θ)° | Relative intensity, % | Peak position (2θ)° | Relative intensity, % |
|---|---|---|---|---|---|
| 4.55 | 100.00 | 18.44 | 13.96 | 24.50 | 16.74 |
| 4.77 | 39.33 | 19.39 | 14.75 | 24.97 | 15.59 |
| 12.65 | 13.49 | 19.58 | 10.27 | 27.33 | 9.99 |
| 13.02 | 60.61 | 21.57 | 13.95 | | |

| | | | | | |
|---|---|---|---|---|---|
| Note: peaks with relative peak intensity >8.0% are listed in the table. | | | | | |

### Example 10: Preparation of Crystal Form III of the compound of formula (C)

Oxalic acid dihydrate (6.3 mg) was weighed and added into a vial, into which a 2-methyltetrahydrofuran solution (40 mg/mL, 0.5 mL) of a sample of Preparation Example 1 was added, and magnetically stirred at room temperature for 3 days. The solid was separated by centrifugation and dried under vacuum at room temperature for 1 day. Upon detection, it was confirmed that a salt was formed, with a a base/acid ratio of 1:1.

A sample was subjected to X-ray powder diffraction, and it was shown to be a crystalline solid (Crystal Form III) and well crystallized. The spectrum is shown in FIG. 10 and its XRPD diffraction peak data are shown in Table 10. A sample was taken for DSC-TGA testing. The DSC pattern showed endothermic peaks at 139.9°C and 204.9°C, and the TGA pattern showed that the sample had a weight loss of 6.20% between room temperature and 150°C.

**Table 10. XRPD diffraction peak data of Crystal Form III of Example 10**

| Peak position (2θ)° | Relative intensity, % | Peak position (2θ)° | Relative intensity, % | Peak position (2θ)° | Relative intensity, % |
|---|---|---|---|---|---|
| 4.55 | 100.00 | 16.71 | 14.80 | 20.41 | 8.49 |
| 4.83 | 54.70 | 17.56 | 9.63 | 21.07 | 9.78 |
| 13.65 | 6.13 | 18.13 | 7.89 | 21.61 | 9.97 |
| 14.01 | 11.46 | 18.72 | 16.95 | 23.28 | 13.37 |
| 16.41 | 9.38 | 19.36 | 16.21 | | |

| | | | | | |
|---|---|---|---|---|---|
| Note: peaks with relative peak intensity >5.0% are listed in the table. | | | | | |

### Example 11: Preparation of Crystal Form IV of the compound of formula (C)

Oxalic acid dihydrate (123.9 mg) and a sample of Preparation Example 1 (503.0 mg) were weighed and added into a reaction flask, into which ethanol (10 mL) was added, and magnetically stirred at room temperature for 2 days. The solid was separated by centrifugation and dried under vacuum at room temperature for 3 days, with a purity of 98.96%. Upon detection, it was confirmed that a salt was formed, with a base/acid ratio of 1:1.

A sample was subjected to X-ray powder diffraction, and it was shown to be a crystalline solid (Crystal Form IV) and well crystallized. The spectrum is shown in FIG. 11 and its XRPD diffraction peak data are shown in Table 11. A sample was taken for DSC-TGA testing. The DSC pattern showed an endothermic peak at 137.4°C, and the TGA pattern showed that the sample had a weight loss of 4.26% between room temperature and 150°C.

**Table 11. XRPD diffraction peak data of Crystal Form IV of Example 11**

| Peak position (2θ)° | Relative intensity, % | Peak position (2θ)° | Relative intensity, % | Peak position (2θ)° | Relative intensity, % |
|---|---|---|---|---|---|
| 4.59 | 100.00 | 18.78 | 2.70 | 20.04 | 3.38 |
| 13.71 | 7.45 | 19.25 | 2.43 | 21.63 | 2.20 |
| 18.11 | 2.92 | 19.49 | 4.26 | 22.93 | 4.68 |
| 18.30 | 2.70 | | | | |

| | | | | | |
|---|---|---|---|---|---|
| Note: peaks with relative peak intensity >2.0% are listed in the table. | | | | | |

### Example 12: Preparation of Crystal Form V of the compound of formula (C)

A sample of Preparation Example 1 (41 mg) and oxalic acid dihydrate (12.9 mg) were weighed in a glass vial, an acetone/water mixed solvent (0.5 mL, v/v=1/1) was added, heated to 80°C and stirred for 12h, and then slowly cooled to room temperature to obtain a single crystal of the compound of formula (C) (acid/base ratio: 1:1). Upon Cu-K_{α} radiation, the resulting single crystal was attributed to a tri-oblique crystal system, a P1 space group, with the following unit cell parameters: {*a*=5.55690 (10) Å, b=16.9102(2) Å, c=18.9473 (2) Å, a=99.1280(10)°, *β*=90.1780(10)°, *γ*=95.1340(10)°, *V*=1750.57 (4) Å³}.

### Example 13: Preparation of other organic acid addition salts of the compound of formula (A)

Samples from Preparation Example 1 was dissolved in each of the solvents shown in the following table to prepare solutions with a concentration of 40 mg/mL; different acids were weighed and respectively added into a vial, and then the prepared sample solution of Preparation Example 1 (0.5 mL) was added. The specific test methods are shown in the following table. The solids were separated by centrifugation and dried under vacuum at room temperature for 1 day.

Upon NMR or HPLC content determination, the samples of Preparation Example 1 could form salts with all the acids listed in the following table. The alkali/acid ratio was 1:1, and a solid could be obtained upon separation. Further, samples were taken for X-ray powder diffraction and the specific results are described in detail in the following table.

**Table 12. Preparation and results of other organic acid addition salts**

| Example | Acid | Amount | Solvent | Reaction condition and test method | Result |
|---|---|---|---|---|---|
| 13-1 | Succinic acid | 4.8 mg | Ethanol | Stirring at room temperature for 4 days, and then stirring at 5°C for 3 days | Crystal |
| 13-2 | Succinic acid | 4.6 mg | Ethyl acetate | Stirring at room temperature for 3 days | Crystal |
| 13-3 | Succinic acid | 4.7 mg | 2-methyl tetrahydrofuran | Stirring at room temperature for 3 days | Crystal |
| 13-4 | Adipic acid | 5.8 mg | Ethyl acetate | Stirring at room temperature for 4 days, and then stirring at 5°C for 3 days | Crystal |
| 13-5 | Tartaric acid | 5.9 mg | Ethanol | Stirring at room temperature for 3 days | Crystal |
| 13-6 | Tartaric acid | 5.9 mg | Acetone/water (19/1, v/v) | Stirring at room temperature for 3 days | Crystal |
| 13-7 | Citric Acid | 7.8 mg | Ethanol | Stirring at room temperature for 3 days | Crystal |
| 13-8 | Citric Acid | 7.7 mg | Acetone/water (19/1, v/v) | Stirring at room temperature for 3 days | Crystal |
| 13-9 | Maleic acid | 4.6 mg | 2-methyl tetrahydrofuran | Stirring at room temperature for 3 days | Solid, amorphous |
| 13-10 | Fumaric acid | 4.5 mg | 2-methyl tetrahydrofuran | Stirring at room temperature for 3 days | Solid, amorphous |
| 13-11 | L-lactic acid | 4.0 mg | Ethyl acetate | Stirring at room temperature for 4 days, and then stirring at 5°C for 3 days | Solid, amorphous |
| 13-12 | Gentisic acid | 6.1 mg | 2-methyl tetrahydrofuran | Stirring at room temperature for 4 days, and then stirring at 5°C for 3 days | Solid, amorphous |
| 13-13 | Gluconic acid | 18.0 mg (50% solution) | Acetone/water (19/1, v/v) | Stirring at room temperature for 4 days, and then stirring at 5°C for 3 days | Crystal |

### Example 14: Preparation of inorganic acid addition salts of the compound of formula (A)

The following salts were prepared by the same method as in Example 13, and the specific results are shown in the following table:

**Table 13. Preparation and results of inorganic acid addition salts**

| Example | Acid | Amount | Solvent | Reaction condition and test method | Result |
|---|---|---|---|---|---|
| 14-1 | Phosphoric Acid | 3 µL (85%) | Ethanol | Stirring at room temperature for 3 days | Crystal |
| 14-2 | Phosphoric Acid | 3 µL (85%) | 2-methyl tetrahydrofuran | Stirring at room temperature for 3 days | Crystal |
| 14-3 | Sulfuric Acid | 10 µL (4M) | Acetone/water (19/1, v/v) | Stirring at room temperature for 4 days, and then stirring at 5°C for 3 days | Crystal |
| 14-4 | Sulfuric Acid | 10 µL (4M) | Ethanol | Stirring at room temperature for 4 days, and then stirring at 5°C for 3 days | Amorphous |

### Test Example 1: Solid stability test of different crystal forms of the compound of formula (B) under different humidity conditions

A proper amount of samples of Example 2 (Crystal Form II of the compound of formula (B)), Example 3 (Crystal Form III of the compound of formula (B)) and Example 4 (Crystal Form IV of the compound of formula (B)) were weighed and placed in vials at 23°C/33% RH and 25°C/60% RH for 7 days, respectively. The samples were subjected to X-ray powder diffraction to investigate the stability of the samples under different conditions, and the results are shown in the following table.

**Table 14. Test results of solid stability of different crystal forms**

| Initial Sample | Condition | Result |
|---|---|---|
| Example 2 (Crystal Form II) | 25 °C/ 60% RH | Crystal Form II |
| Example 3 (Crystal Form III) | | Crystal Form III |
| Example 4 (Crystal Form IV) | | Crystal Form IV |
| Example 2 (Crystal Form II) | 23 °C/ 33% RH | Crystal Form II |
| Example 3 (Crystal Form III) | | Crystal Form III |
| Example 4 (Crystal Form IV) | | Crystal Form IV |

Conclusion: As Crystal Form II of Example 2, Crystal Form III of Example 3 and Crystal Form IV of Example 4 were placed under different humidity conditions, the crystal forms were kept unchanged.

### Test Example 2: Solid stability test of different crystal forms of the compound of formula (B)

A proper amount of samples of Example 2 (Crystal Form II of the compound of formula (B)) and Example 5 (Crystal Form V of the compound of formula (B)) were weighed and placed in vials at a high temperature (60°C, sealed) for 7 days. The samples were subjected to X-ray powder diffraction to investigate the stability of the samples under different conditions, and the results are shown in the following table.

**Table 15. Solid stability test results of different crystal forms of the compound of formula (B)**

| Initial Sample | Condition | Result |
|---|---|---|
| Example 2 (Crystal Form II) | 60°C, sealed | Crystal Form II |
| Example 5 (Crystal Form V) | | Crystal Form V |

Conclusion: As Crystal Form II of Example 2 and Crystal Form V of Example 5 were placed at high temperature for 7 days, the crystal forms were kept stable.

### Test Example 3: Long-term (three months) stability test of Crystal Form I of the compound of formula (B)

A proper amount of the sample of Example 1 (Crystal Form I of the compound of formula (B)) was weighed in a vial for investigation of long-term stability, and the results are shown in the following table.

**Table 16. Long-term (three months) stability test results of Crystal Form I of the compound of formula (B)**

| Initial Sample | Initial purity | Time (month) | Result | |
|---|---|---|---|---|
| | | | Purity | Crystal Form |
| Example 1 (Crystal Form I) | 99.43% | 3 | 99.32% | Crystal Form I |

Conclusion: Crystal Form I of Example 1 was kept substantially unchanged in purity and the crystal form was unchanged in a long-term experiment for 3 months.

### Test Example 4: Solubility Test

The salts of the compound of formula (A) obtained in the above examples have good solubility in water and biological vehicle media, and representative examples are shown below.

The solubility of Example 7 (the compound of formula (B), Crystal Form VII), Example 10 (the compound of formula (C), Crystal Form) and a sample in a free form (Preparation Example 1) was tested in water, SGF and FassIF at 37°C for 24 hours, and the results are shown in the following table.

| Sample | 24 h solubility (mg/mL) | | |
|---|---|---|---|
| | Water | SGF | FassIF |
| Malate (Sample of Example 7) | >4.9 | >4.8 | >4.7 |
| Oxalate (Sample of Example 11) | >9.1 | >9.2 | >9.1 |
| Free alkali (Preparation Example 1) | 0.39 | >4.4 | 1.7 |

| | | | |
|---|---|---|---|
| Note: (1) solubility is in free state; (2) > indicates that the added sample is completely dissolved without increasing the sample amount. | | | |

The solubility of the Crystal Form VII of the compound of formula (B) and the Crystal Form IV of the compound of formula (C) in water and FassIF media was significantly higher than the free base sample obtained in Preparation Example 1.

### Experimental Example 1: Experiment on in vitro efficacy

### 1. Enzymatic experiment

The radiometric assay for PRMT5 based on FlashPlate technology was adopted to determine IC₅₀ of the test compound.

After dissolved in dimethyl sulfoxide respectively, the test compounds were added into an Echo Qualified 384-well plate and diluted to the desired concentrations. The diluted test compounds were transferred from the Echo Qualified 384-well plate to a 384-well reaction plate using an Echo 550 instrument, and dimethyl sulfoxide was transferred into both the control and blank wells. PRMT5 was added to 1X reaction buffer (including 10 mM Tris-HCl; pH 8.0; 0.01% Tween-20; 1 mM DTT) to form a 1.67X enzyme solution (at an enzyme concentration of 5 nM). A polypeptide substrate and [³H]-SAM were added to 1X reaction buffer to form a 2.5X substrate solution (the terminal concentrations of the substrates were 100 nM and 250 nM, respectively). At a volume of 15 µL/well, the 1.67X enzyme solution was added into wells of the 384-well reaction plate. In case of the blank wells, the enzyme solution was replaced with 15 µL of the 1X reaction buffer. The reaction plate was centrifuged at 1000 rpm for 1 min, and incubated at room temperature for 15 min. To each well of the 384-well reaction plate, 10 µL of the 2.5X substrate solution was added, centrifuged at 1000 rpm for 1 min, and reacted at 25°C for 60 min. To each well of the 384-well reaction plate, 5 µL of reaction stop solution (which was 125 µM cold SAM solution) was added to terminate the reaction. From each well of the test plate, 25 µL was measured and transferred to Flashplate and left at room temperature for 1 h. Thereafter, the Flashplate was washed with 0.1% Tween-20 solution three times. Readings were taken with MicroBeta 2. The data was converted into the inhibition rate data. Inhibition rate = (conversion rate _{control well} - conversion rate _{compound well}) / (conversion rate _{control well} - conversion rate _{blank well}) × 100%. The IC₅₀ curves were fitted using XLFit 5.4.0.8. The fitting equation was as follows: Y = Bottom + (Top-Bottom) / (1 + (IC₅₀/X)^HillSlope).

### 2. Cell experiment

The complete medium required for the culture of human acute monocytic leukemia cell line MV4-11 (Shanghai Cell Bank) for experimentwas INMM (CatNO. 12440-053, gibco) supplemented with 10% FBS (Cat NO. SA311.02, cellmax). The cells were cultured in an incubator at 37°C with 5% CO₂. The experiment reagents included dimethyl sulfoxide (Tianjin Kemiou Chemical Reagent Co., Ltd.) and MTT (THIAZOLYL BLUE TETRAZOLIUM BROMIDE, CAS. NO. 298-93-1, VWR). The control compound GSK3326595 for experiment was homemade or commercially available. The test compound was encapsulated and stored at 4°C.

Dimethyl sulfoxide was used as a vehicle to fully dissolve the test compound, thereby formulating a stock solution at a concentration of 5×10⁻² mol/L. The stock solution was stored at - 20°C. With the complete medium as diluent, the test compound was subjected to gradient dilution to afford dilutions at different concentrations for standby application. In a 96-well culture plate, the suspension of the human acute monocytic leukemia cell line MV4-11 in the complete medium, at a volume of 100 µL/well (2×10³ cells/well), was added. Afterwards, the dilutions at different concentrations of the corresponding test compounds (100 µL/well) were added, respectively. Each of the test compounds was provided with 8 concentration gradients and three replicates were used for each concentration. The plates were incubated in an incubator at 37°C with 5% CO₂. On Day 6, the plate was treated with MTT (20 µL/well) and incubated for 4 h in an incubator at 37°C with 5% CO₂. The supernatant was discarded. Dimethyl sulfoxide (150 µL/well) was added and mixed well by shaking. The OD value at 550 nm was measured with a microplate reader. Wells containing only cell suspensions without the test compounds were control wells, and wells containing only complete media were blank wells. The cell growth inhibition rate was calculated according to the following equation:
Inhibition rate = (OD value _{control well} - OD value _{sample well}) / (OD value _{control well} - OD value _{blank well}) × 100%. According to the inhibition rate at each concentration, the half maximal inhibitory concentration IC₅₀ value was calculated using the SPSS software. The results were listed in Table 18.

**Table 17. Experimental data for enzymatic effects**

| Test Compound | Enzymology (IC₅₀, nM) |
|---|---|
| GSK3326595 | 47 |
| Compound of formula (A) | 31 |

**Table 18. Experimental data for cytological effects**

| Test Compound | MV4-11 MTT (IC₅₀, nM) |
|---|---|
| GSK3326595 | 6.683 |
| Compound of formula (A) | 6.767 |

The enzymatic and cytological screening results showed that the compound of formula (A) of the present disclosure exhibited better inhibitory activities at both enzyme and cell levels, as compared to the positive control drug GSK3326595 (the 208^{th} compound in WO2014100719).

### Test Example 2: Experiment on in vivo efficacy

Experimental female NOD-SCID mice, SPF grade, 4 to 5 weeks, were purchased from Beijing Vital River Laboratory Animal Technology Co., Ltd. The mice were intraperitoneally injected with cyclophosphamide at a dose of 100 mg/kg one day before cell inoculation. The human acute monocytic leukemia cell line MV4-11 (1 × 10⁷/0.1 ml/mouse) was subcutaneously inoculated into forelimb axilla of mice to establish a subcutaneous xenograft model. When the tumor volumes reached about 110 mm³ (10 days after inoculation), the mice were equally grouped according to the tumor volume, with 5 mice in each group, i.e., vehicle group (2% DMSO + 98% (0.2 g/mL) hydroxypropyl-β-cyclodextrin) and test drug groups. For the test drug groups, the dose of administration was 100 mg/kg, the dosing volume was 10 mL/kg, the dosing frequency was BID, the tumor diameters were measured twice per week, and the data was recorded. The mice were administered for 11 consecutive days. When the experiment ended, the tumors were dissected and weighed.

The body weight growth rate, the tumor volume, and the tumor weight inhibition rate were calculated according to the following equations: body weight growth rate = X_{(Wi-W0)} / X_{W0} × 100%, where Wᵢ denoted the body weight of a mouse in each test drug group on the n^{th} day, and W₀ denoted the body weight of a mouse in each test drug group at the beginning of administration; tumor volume (V) = 1/2 × a × b², where a and b denoted the long diameter and the short diameter of a tumor, respectively; d0 denoted before grouping for administration, d9 denoted the 9^{th} day after administration, relative tumor volume (RTV) = d9 tumor volume / d0 tumor volume; tumor weight inhibition rate = (tumor weight _{vehicle group} - tumor weight _{test drug group}) / tumor weight _{vehicle group} × 100%. The results were listed in Table 19.

**Table 19. MV4-11 In vivo experimental data**

| Drug | Dose (mg/kg) | Animal number (piece) | Body Weight Change (%) | d0 Tumor Volume (mm³) | d9 Tumor Volume (mm³) | RTV (d9) | Tumor Weight (g) | Tumor weight inhibition rate (%) |
|---|---|---|---|---|---|---|---|---|
| Vehicle group | 0 | (4/5) | 6.8 | 164.5±29. 1 | 575.1± 205.3 | 3.7± 1.7 | 0.7265± 0.2458 | 0.0 |
| GSK3326595 | 100 | (5/5) | -6.8 | 165.2±23. 1 | 135± 43** | 0.8± 0.2** | 0.1124± 0.0764* ** | 84.5 |
| Compound of formula (A) | 100 | (5/5) | -6.1 | 165.3±27. 5 | 55.7± 19.7***## | 0.4± 0.1 ** ## | 0.0648± 0.0267* **### | 91.1 |

| | | | | | | | | |
|---|---|---|---|---|---|---|---|---|
| Note: Compared with the vehicle group, *p<0.05, ***p*<0.01, ****p*<0.001; compared with the control drug GSK3326595, ##p<0.01, *###p*<0.001*.* | | | | | | | | |

The experimental results of the MV4-11 *in vivo* efficacy in MV4-11 xenografts model showed that the compound of formula (A) of the present disclosure had a significant tumor-inhibitory activity, and had significantly better inhibitory effects on the tumor volume and tumor weight than those of the control drug GSK3326595.

### Test Example 3: Pharmacokinetic experiment

SD rats (male, 180 g to 200 g, Beijing Vital River Laboratory Animal Technology Co., Ltd.) were administered with the test compounds (solvent: 2% DMSO + 98% (0.2 g/mL) hydroxypropyl-β-cyclodextrin, dosing volume: 5 mL/kg) by gavage. Blood was collected from the orbits of the rats at different points in time (0.25, 0.5, 1, 2, 4, 8, 24 h) after administration. The collected whole blood was anticoagulated with heparin sodium and centrifuged at 3000 g to obtain rat plasma samples, and methanol was added thereto to precipitate proteins. The drug concentration in rat plasma after administration was determined by HPLC-MS/MS method. A drug concentration-time curve was plotted and the pharmacokinetic parameters were calculated. Pharmacokinetic behaviors of the compounds after administration in rats were described by means of the parameter estimation of non-compartmental model based on statistical moment theory. The results were listed in Table 20.

**Table 20. PK experimental data in SD rats**

| Drug | Dose (mg/kg) | Tₘₐₓ (hr) | Cₘₐₓ (ng/mL) | AUC_{0-24 h} (h*ng/mL) |
|---|---|---|---|---|
| GSK3326595 | 25 | 2.67 | 60.3 | 589 |
| Compound of formula (A) | 25 | 0.333 | 2370 | 4406 |

The results of the PK experiment suggested that the compound of formula (A) of the present disclosure were all better absorbed in rats with a shorter time to peak, a higher peak concentration, and a significantly increased *in vivo* exposure as compared to the positive control drug GSK3326595.

While the foregoing invention has been described in considerable details by way of illustration and examples for the purpose of a clear understanding, it will be apparent to those skilled in the art from the teachings of this disclosure that certain changes and modifications may be made thereto without departing from the spirit or scope of the appended claims.

## Claims

1. A compound which is an inorganic acid addition salt or an organic acid addition salt of a compound represented by formula (A):

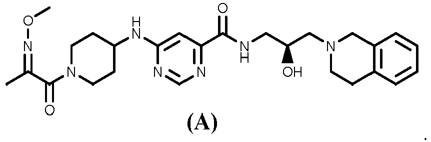

2. The compound according to claim 1, wherein the inorganic acid addition salt is a sulfate or phosphate.

3. The compound according to claim 1, wherein the organic acid addition salt is selected from the group consisting of a malate, an oxalate, a succinate, a tartrate, an adipate, a citrate, a gluconate, a maleate, a fumarate, a lactate and a gentisate.

4. The compound according to claim 1, wherein in the inorganic acid addition salt or the organic acid addition salt of the compound represented by formula (A), the stoichiometric ratio of the compound represented by formula (A) to the organic acid or inorganic acid molecule is 1:0.5-2; preferably 1:1-1.5; further preferably 1:1, 1:1.1, 1:1.2, 1:1.3, 1:1.4 or 1:1.5; further preferably 1:1, 1:1.3, 1:1.4 or 1:1.5; further preferably 1:1.

5. The compound according to claim 1, wherein the compound is a compound represented by formula (B): 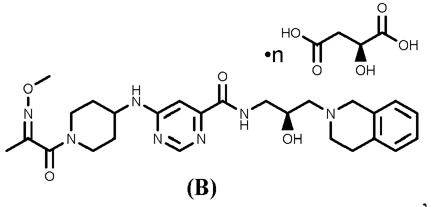 wherein n is from 0.5 to 2; preferably 1 to 1.5; further preferably 1, 1.1, 1.2, 1.3, 1.4 or 1.5; still further preferably 1 or 1.5.

6. The compound according to claim 5, wherein the compound is Crystal Form I of the compound represented by formula (B) which, by Cu-Kα radiation, has an X-ray powder diffraction pattern that comprises characteristic diffraction peaks at 2θ angles of 4.2±0.2°, 6.5±0.2°, 13.3±0.2°, and 19.1±0.2°.

7. The compound according to claim 5, wherein the compound is Crystal Form II of the compound represented by formula (B) which, by Cu-K_{α} radiation, has an X-ray powder diffraction pattern that comprises characteristic diffraction peaks at 2θ angles of 4.9±0.2°, 6.6±0.2°, 13.2±0.2°, 18.7±0.2°, and 19.8±0.2°.

8. The compound according to claim 5, wherein the compound is Crystal Form III of the compound represented by formula (B) which, by Cu-K_{α} radiation, has an X-ray powder diffraction pattern that comprises characteristic diffraction peaks at 2θ angles of 4.3±0.2°, 6.9±0.2°, and 20.3±0.2°.

9. The compound according to claim 5, wherein the compound is Crystal Form IV of the compound represented by formula (B) which, by Cu-K_{α} radiation, has an X-ray powder diffraction pattern that comprises characteristic diffraction peaks at 2θ angles of 4.4±0.2°, 7.6±0.2°, 8.9±0.2°, 13.9±0.2°, and 20.6±0.2°.

10. The compound according to claim 5, wherein the compound is Crystal Form V of the compound represented by formula (B) which, by Cu-K_{α} radiation, has an X-ray powder diffraction pattern that comprises characteristic diffraction peaks at 2θ angles of 5.0±0.2°, 13.6±0.2°, 18.6±0.2°, 19.6±0.2°, and 20.2±0.2°.

11. The compound according to claim 5, wherein the compound is Crystal Form VI of the compound represented by formula (B) which, by Cu-K_{α} radiation, has an X-ray powder diffraction pattern that comprises characteristic diffraction peaks at 2θ angles of 4.5±0.2°, 7.0±0.2°, 9.0±0.2°, 12.9±0.2°, 20.2±0.2°, and 21.6±0.2°.

12. The compound according to claim 5, wherein the compound is Crystal Form VII of the compound represented by formula (B) which, by Cu-Kα radiation, has an X-ray powder diffraction pattern that comprises characteristic diffraction peaks at 2θ angles of 4.3±0.2°, 6.9±0.2°, 13.2±0.2°, 19.1±0.2°, and 20.0±0.2°.

13. The compound according to claim 1, wherein the compound is a compound represented by formula (C): 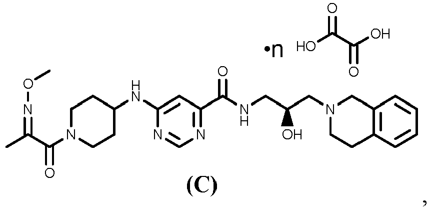 wherein n is from 0.5 to 2; preferably 1 to 1.5; further preferably 1, 1.1, 1.2, 1.3, 1.4 or 1.5; still further preferably 1 or 1.5.

14. The compound according to claim 13, wherein the compound is Crystal Form I of the compound represented by formula (C) which, by Cu-Kα radiation, has an X-ray powder diffraction pattern that comprises characteristic diffraction peaks at 2θ angles of 4.7±0.2°, 7.1±0.2°, 10.7±0.2°, 17.4±0.2°, and 21.2±0.2°.

15. The compound according to claim 13, wherein the compound is Crystal Form II of the compound represented by formula (C) which, by Cu-Kα radiation, has an X-ray powder diffraction pattern that comprises characteristic diffraction peaks at 2θ angles of 4.6±0.2°, 13.0±0.2°, and 21.6±0.2°.

16. The compound according to claim 13, wherein the compound is Crystal Form III of the compound represented by formula (C) which, by Cu-Kα radiation, has an X-ray powder diffraction pattern that comprises characteristic diffraction peaks at 2θ angles of 4.6±0.2°, 18.7±0.2°, and 19.4±0.2°.

17. The compound according to claim 13, wherein the compound is Crystal Form IV of the compound represented by formula (C) which, by Cu-Kα radiation, has an X-ray powder diffraction pattern that comprises characteristic diffraction peaks at 2θ angles of 4.6±0.2°, 13.7±0.2°, 19.5±0.2°, 20.0±0.2°, and 22.9±0.2°.

18. A pharmaceutical composition comprising the compound according to any one of claims 1 to 17; preferably, wherein the pharmaceutical composition further comprises a pharmaceutically acceptable carrier.

19. Use of the compound according to any one of claims 1 to 17 or the pharmaceutical composition according to claim 18 in the preparation of a medicament.

20. The use according to claim 19, wherein the medicament is for preventing and/or treating a cell proliferative disease; preferably, the cell proliferative disease is a tumor or cancer; more preferably, the tumor or cancer is a hematological neoplasm or a solid tumor, further preferably a malignant hematological neoplasm or an advanced solid tumor, and still further preferably a relapsed or refractory hematological neoplasm or advanced malignant solid tumor.

21. The use according to claim 19, wherein the medicament is for preventing and/or treating diseases mediated at least partially by PRMT5.
